# EUROPEAN PATENT APPLICATION

(11) **EP 3 835 414 A1**
(43) Date of publication of application: **16.06.2021**
(21) Application number: 19848673.0
(22) Date of filing: 06.08.2019
(51) Int. Cl.: C12N 5/071, C12N 5/0783, A61K 38/17, A61P 35/00, A61P 31/00

(54) **METHOD FOR ACTIVATING CD4+T CELL**

(30) Priority: 07.08.2018 CN 201810892647
(71) Applicant: INSTITUTE OF BIOPHYSICS, CHINESE ACADEMY OF SCIENCES, Chaoyang District Beijing 100101 (CN); INSTITUT PASTEUR OF SHANGHAI, CHINESE ACADEMY OF SCIENCES, Life Sciences Research Building Shanghai 200031 (CN)
(72) Inventor: HOU, Baidong, Beijing 100101 (CN); HONG, Sheng, Beijing 100101 (CN); HUA, Zhaolin, Beijing 100101 (CN); TANG, Hong, Shanghai 200031 (CN)
(74) Representative: Lasar, Andrea Gisela
(86) International application number: PCT/CN2019/099489
(87) International publication number: WO 2020/029968

(57) **Abstract**

Provided is a method for activating CD4+ T cells using a polymer-based antigen complex. The method comprises the steps of bringing the polymer-based antigen complex into contact with B cells so that B cells process and present the antigen complex, and of bringing the B cells into contact with CD4+ T cells to activate CD4+ T cells. Also provided are a method for promoting the differentiation of CD4+ T cells into Tfh cells and Th1 cells using the antigen complex, and a method for treating diseases by activating CD4+ T cells and/or promoting the differentiation of CD4+ T cells.

SEQUENCE LISTING

<110>  INSTITUTE OF BIOPHYSCIS, CHINESE ACADEMY OF SCIENCES
<110>  INSTITUT PASTEUR OF SHANGHAI, CHINESE ACADEMY OF SCIENCES

<120>  METHOD FOR ACTIVATING CD4+ T CELL

<130>  I09680WOEP/LA

<140>  EP 19 848 673.0
<141>  2019-08-06

<150>  CN 201810892647.8
<151>  2018-08-07

<160>  3

<170>  PatentIn version 3.5

<210>  1
<211>  133
<212>  PRT
<213>  Phage Qbeta

<400>  1

Met Ala Lys Leu Glu Thr Val Thr Leu Gly Asn Ile Gly Lys Asp Gly
1               5                   10                  15

Lys Gln Thr Leu Val Leu Asn Pro Arg Gly Val Asn Pro Thr Asn Gly
            20                  25                  30

Val Ala Ser Leu Ser Gln Ala Gly Ala Val Pro Ala Leu Glu Lys Arg
        35                  40                  45

Val Thr Val Ser Val Ser Gln Pro Ser Arg Asn Arg Lys Asn Tyr Lys
    50                  55                  60

Val Gln Val Lys Ile Gln Asn Pro Thr Ala Cys Thr Ala Asn Gly Ser
65                  70                  75                  80

Cys Asp Pro Ser Val Thr Arg Gln Ala Tyr Ala Asp Val Thr Phe Ser
                85                  90                  95

Phe Thr Gln Tyr Ser Thr Asp Glu Glu Arg Ala Phe Val Arg Thr Glu
            100                 105                 110

Leu Ala Ala Leu Leu Ala Ser Pro Leu Leu Ile Asp Ala Ile Asp Gln
        115                 120                 125

Leu Asn Pro Ala Tyr
    130

<210>  2

<211>  185
<212>  PRT
<213>  Hepatitis B virus

<400>  2

Met Asp Ile Asp Pro Tyr Lys Glu Phe Gly Ala Thr Val Glu Leu Leu
1               5                   10                  15

Ser Phe Leu Pro Ser Asp Phe Phe Pro Ser Val Arg Asp Leu Leu Asp
            20                  25                  30

Thr Ala Ser Ala Leu Tyr Arg Glu Ala Leu Glu Ser Pro Glu His Cys
        35                  40                  45

Ser Pro His His Thr Ala Leu Arg Gln Ala Ile Leu Cys Trp Gly Glu
    50                  55                  60

Leu Met Thr Leu Ala Thr Trp Val Gly Asn Asn Leu Glu Asp Pro Ala
65                  70                  75                  80

Ser Arg Asp Leu Val Val Asn Tyr Val Asn Thr Asn Met Gly Leu Lys
                85                  90                  95

Ile Arg Gln Leu Leu Trp Phe His Ile Ser Cys Leu Thr Phe Gly Arg
            100                 105                 110

Glu Thr Val Leu Glu Tyr Leu Val Ser Phe Gly Val Trp Ile Arg Thr
        115                 120                 125

Pro Pro Ala Tyr Arg Pro Pro Asn Ala Pro Ile Leu Ser Thr Leu Pro
    130                 135                 140

Glu Thr Thr Val Val Arg Arg Arg Asp Arg Gly Arg Ser Pro Arg Arg
145                 150                 155                 160

Arg Thr Pro Ser Pro Arg Arg Arg Arg Ser Gln Ser Pro Arg Arg Arg
                165                 170                 175

Arg Ser Gln Ser Arg Glu Ser Gln Cys
            180                 185


<210>  3
<211>  131
<212>  PRT
<213>  Phage AP205

<400>  3

Met Ala Asn Lys Pro Met Gln Pro Ile Thr Ser Thr Ala Asn Lys Ile
1               5                   10                  15

Val Trp Ser Asp Pro Thr Arg Leu Ser Thr Thr Phe Ser Ala Ser Leu
            20                  25                  30

Leu Arg Gln Arg Val Lys Val Gly Ile Ala Glu Leu Asn Asn Val Ser
        35                  40                  45

Gly Gln Tyr Val Ser Val Tyr Lys Arg Pro Ala Pro Lys Pro Glu Gly
    50                  55                  60

Cys Ala Asp Ala Cys Val Ile Met Pro Asn Glu Asn Gln Ser Ile Arg
65                  70                  75                  80

Thr Val Ile Ser Gly Ser Ala Glu Asn Leu Ala Thr Leu Lys Ala Glu
                85                  90                  95

Trp Glu Thr His Lys Arg Asn Val Asp Thr Leu Phe Ala Ser Gly Asn
            100                 105                 110

Ala Gly Leu Gly Phe Leu Asp Pro Thr Ala Ala Ile Val Ser Ser Asp
        115                 120                 125

Thr Thr Ala
    130

## Description

This application claims the priority of Chinese Patent Application No. CN 201810892647.8 filed on August 7, 2018, content of which is incorporated herein as a part of this application by reference in its entirety.

### TECHNICAL FIELD

The present disclosure relates to the field of immunotherapy. In particular, the present disclosure relates to a method for activating CD4+ T cells using a multimer-based antigen complex. The present disclosure also relates to a method for promoting differentiation of CD4+ T cells into Tfh cells and Th1 cells, and a method for treating a disease by activating CD4+ T cells and/or promoting differentiation of CD4+ T cells.

### BACKGROUND

CD4+ T cells are an important type of T lymphocytes, which play an important role in a variety of physiological and pathological processes, including infection-related immune responses, tumor-related immune responses, allergic disease-related immune responses, immune responses in autoimmune diseases and the like. A main function of CD4+ T cells is to act by regulating other immune cells, including regulating functions of B lymphocytes, CD8+ T lymphocytes, mononuclear macrophages, NK cells and other immune cells in adaptive immune responses and natural immune responses.

CD4+ T cells may be categorized into several different subpopulations according to their functional status, each of which acts specifically in various immune responses. Follicular helper T cells (Tfhs) are an important subset of CD4+ T cells, which are mainly involved in the process of germinal center reaction. Tfhs are essential for formation and maintenance of a germinal center. By inducing and maintaining the effects of germinal center B cells, Tfhs may promote various effects such as antibody production, conversion of antibody types, antibody affinity maturation, neutralizing antibody production, and increase in the breadth of the antibody profile. Therefore, how to efficiently produce Tfh cells is a key link in the research and development of a variety of vaccines. Type 1 helper T cells (Th1) are another important subset of CD4+ T cells, which play a key role in antiviral and antibacterial responses, especially those against an intracellular bacterial infection, such as a tuberculosis infection. In addition, CD4+ T cells also play an important role in anti-tumor immunity.

To perform the above functions, it is essential for CD4+ T cells to first transform from a resting state to an activated cell state. The current method for activating CD4+ T cells mainly utilizes dendritic immune cells (DC) to initially activate the CD4+ T cells in vivo or in vitro, thereby achieving the purpose of generating activated CD4+ T cells. However, the activation pathway of CD4+ T cells and related mechanism thereof are still unclear, and DCs are not specific for antigen processing and presentation. It is still desirable for a new method for effectively activating CD4+ T cells.

### SUMMARY

The present disclosure is based at least in part on the discovery that by constructing a multimer-based antigen complex, it may be recognized and processed by B cells, thereby activating the CD4+ T cells and promoting the differentiation of the CD4+ T cells into Tfh and Th1 cells. As B cells specifically recognize an antigen through a B cell surface receptor encoded by immunoglobulin receptor genes, a stronger effect may be achieved in activation of CD4+ T cells by using B cells than that when DCs are used.

Accordingly, the present disclosure relates to the following aspects.

In an aspect, the present disclosure relates to a method for activating CD4+ T cells, including the following steps:
a) contacting a multimer-based antigen complex with a population of B cells,
   the antigen complex comprising:
   i) a multimer assembled from a plurality of subunits; and
   ii) an immunostimulant,
   wherein the plurality of subunits comprise or consist of a target antigen, and wherein the immunostimulant is packaged in the multimer, or attached to the multimer by physical adsorption or chemical linkage;
   alternatively, the antigen complex comprising:
   i) a multimer assembled from a plurality of subunits;
   ii) a loaded target antigen; and
   iii) an immunostimulant,
   wherein the target antigen is attached to the surface of the multimer by physical adsorption or chemical linkage, or fused with at least a part of the plurality of subunits by gene fusion, which fusion does not affect the assembly of the multimer, and the target antigen is displayed on the surface of the multimer after the multimer is assembled; and wherein the immunostimulant is packaged in the multimer, or attached to the multimer by physical adsorption or chemical linkage,
   wherein at least a part of the population of B cells are capable of recognizing at least one of the subunits;
b) incubating the antigen complex with the population of B cells to allow B cells to recognize and process the antigen complex, and present the target antigen on the cell surface; and
c) contacting the population of B cells with CD4+ T cells to activate the CD4+ T cells.

In another aspect, the present disclosure relates to a method for promoting differentiation of CD4+ T cells into follicular helper T cells (Tfh) and/or helper T cells 1 (Th1), comprising the following steps:
a) contacting a multimer-based antigen complex with a population of B cells,
   the antigen complex comprising:
   i) a multimer assembled from a plurality of subunits; and
   ii) an immunostimulant,
   wherein the plurality of subunits comprise or consist of a target antigen, and wherein the immunostimulant is packaged in the multimer, or attached to the multimer by physical adsorption or chemical linkage;
   alternatively, the antigen complex comprising:
   i) a multimer assembled from a plurality of subunits;
   ii) a loaded target antigen; and
   iii) an immunostimulant,
   wherein the target antigen is attached to the surface of the multimer by physical adsorption or chemical action, or fused with at least a part of the plurality of subunits by gene fusion, which fusion does not affect the assembly of the multimer, and the target antigen is displayed on the surface of the multimer after the multimer is assembled; and wherein the immunostimulant is packaged in the multimer, or attached to the multimer by physical adsorption or chemical action,
   wherein at least a part of the population of B cells are capable of recognizing at least one of the subunits;
b) incubating the antigen complex with the population of B cells to allow B cells to recognize and process the antigen complex, and present the target antigen on the cell surface;
c) contacting the B cells with CD4+ T cells to promote differentiation of the CD4+ T cells into Tfh and/or Th1.

In some embodiments of the method for activating T cells and the method for promoting differentiation of CD4+ T cells into Tfh and/or Th1 cells described above, the population of B cells may be a population of B cells isolated from peripheral blood or a lymphoid organ of a donor.

In some embodiments of the method for activating T cells and the method for promoting differentiation of CD4+ T cells into Tfh and/or Th1 cells described above, after step b), the method may further comprise a step of screening, enriching and/or amplifying the B cells that recognize the subunit.

In some embodiments of the method for activating T cells and the method for promoting differentiation of CD4+ T cells into Tfh and/or Th1 cells described above, after step b), the method may further comprise a step of screening the B cells that recognize the subunit, and introducing a gene sequence encoding an immunoglobulin receptor into the population of B cells, to increase the number of B cells that recognize the subunit in the population.

In any embodiment of the method for activating T cells and the method for promoting differentiation of CD4+ T cells into Tfh and/or Th1 cells described above, the multimer has a diameter of about 10 nm to about 1000 nm.

In any embodiment of the method for activating T cells and the method for promoting differentiation of CD4+ T cells into Tfh and/or Th1 cells described above, the multimer may comprise at least 4 subunits.

In any embodiment of the method for activating T cells and the method for promoting differentiation of CD4+ T cells into Tfh and/or Th1 cells described above, the immunostimulant may be selected from a bacteria-derived ssRNA, an artificially synthesized ssRNA or a derivative thereof, an artificially synthesized CpG-containing oligonucleotide, an interferon, a cytokine, and a combination thereof. In some embodiments, the bacteria-derived ssRNA is an E. coli-derived ssRNA. In some embodiments, the interferon is selected from type I interferon, type II interferon, type III interferon, and a combination thereof. In some embodiments, the cytokine is selected from IL-6, IL-12, IL21, and a combination thereof.

In some embodiments of the method for activating T cells and the method for promoting differentiation of CD4+ T cells into Tfh and/or Th1 cells described above, the multimer is a virus-like particle. In some embodiments, the virus-like particle comprises or consists of Qβ protein, HBcAg or AP205.

In some embodiments, the virus-like particle comprises or consists of the target antigen. For example, the target antigen is selected from Qβ protein, HBcAg and AP205.

In some embodiments of the method for activating T cells and the method for promoting differentiation of CD4+ T cells into Tfh and/or Th1 cells described above, the antigen complex comprises a loaded target antigen that is a bacteria-derived or virus-derived antigen. In some embodiments, the target antigen is a mycobacterium tuberculosis-derived antigen, for example, selected from crystallin and Rv3133c. In other embodiments, the target antigen is a superbacteria-derived antigen, for example, selected from Klebsiella pneumoniae carbapenemase and penicillin binding protein. In still other embodiments, the target antigen is a lentivirus-derived antigen, for example, selected from HBV pre-S1 antigen and EBV LMP1 antigen.

In other embodiments of the method for activating T cells and the method for promoting differentiation of CD4+ T cells into Tfh and/or Th1 cells described above, the antigen complex comprises a loaded target antigen that is a tumor-associated antigen. In some embodiments, the tumor-associated antigen is selected from Her2, p53 and tumor neoantigen.

In some embodiments of the method for activating T cells and the method for promoting differentiation of CD4+ T cells into Tfh and/or Th1 cells described above, the method is an in vitro method. In other embodiments, step c) of the method occurs in vivo.

In another aspect, the present disclosure relates to a method for preventing and/or treating a disease in a subject in need thereof, comprising:
a) isolating a population of B cells from the subject;
b) contacting a multimer-based antigen complex with the population of B cells;
   the antigen complex comprising:
   i) a multimer assembled from a plurality of subunits; and
   ii) an immunostimulant,
   wherein the plurality of subunits comprise or consist of a target antigen, and wherein the immunostimulant is packaged in the multimer, or attached to the multimer by physical adsorption or chemical linkage;
   alternatively, the antigen complex comprising:
   i) a multimer assembled from a plurality of subunits;
   ii) a loaded target antigen; and
   iii) an immunostimulant,
   wherein the target antigen is attached to the surface of the multimer by physical adsorption or chemical linkage, or fused with at least a part of the plurality of subunits by gene fusion, which fusion does not affect the assembly of the multimer, and the target antigen is displayed on the surface of the multimer after the multimer is assembled; and wherein the immunostimulant is packaged in the multimer, or attached to the multimer by physical adsorption or chemical linkage;
   wherein at least a part of the population of B cells are capable of recognizing at least one of the subunits;
c) incubating the antigen complex with the population of B cells to allow B cells to recognize and process the antigen complex, and present the target antigen on the cell surface;
d) administering the population of B cells to the subject.

In some embodiments of the method for preventing and/or treating a disease described above, the population of B cells is a population of B cells isolated from peripheral blood or a lymphoid organ of the subject.

In some embodiments of the method for preventing and/or treating a disease described above, after step c), the method further comprises a step of screening, enriching and/or amplifying the B cells that recognize the subunit.

In other embodiments of the method for preventing and/or treating a disease described above, after step c), the method further comprises a step of screening the B cells that recognize the subunit, and introducing a gene sequence encoding an immunoglobulin receptor into the population of B cells, to increase the number of B cells that recognize the subunit in the population.

In any embodiment of the method for preventing and/or treating a disease described above, the multimer has a diameter of 10 nm to 1000 nm.

In any embodiment of the method for preventing and/or treating a disease described above, the multimer comprises at least 4 subunits.

In any embodiment of the method for preventing and/or treating a disease described above, the immunostimulant comprises a bacteria-derived ssRNA, an artificially synthesized ssRNA or a derivative thereof, an artificially synthesized CpG-containing oligonucleotide, an interferon, a cytokine, and a combination thereof. In some embodiments, the bacteria-derived ssRNA is an E. coli-derived ssRNA. In some embodiments, the interferon is selected from type I interferon, type II interferon, type III interferon, and a combination thereof. In some embodiments, the cytokine is selected from IL-6, IL-12, IL21, and a combination thereof.

In some embodiments of the method for preventing and/or treating a disease described above, the multimer is a virus-like particle. In some embodiments, the virus-like particle comprises or consists of Qβ protein, HBcAg or AP205.

In some embodiments, the virus-like particle comprises or consists of the target antigen. For example, the target antigen is selected from Qβ protein, HBcAg and AP205.

In some embodiments of the method for preventing and/or treating a disease described above, the disease is an infectious disease, and the antigen complex comprises a loaded target antigen that is a bacteria-derived or virus-derived antigen. In some embodiments, the target antigen is a mycobacterium tuberculosis-derived antigen, for example, selected from crystallin and Rv3133c. In other embodiments, the target antigen is a superbacteria-derived antigen, for example, selected from Klebsiella pneumoniae carbapenemase and penicillin binding protein. In still other embodiments, the target antigen is a lentivirus-derived antigen, for example, selected from HBV pre-S1 antigen and EBV LMP1 antigen.

In other embodiments of the method for preventing and/or treating a disease described above, the disease is a cancer, and the antigen complex comprises a loaded target antigen that is a tumor-associated antigen. In some embodiments, the tumor-associated antigen is selected from Her2, p53 and tumor neoantigen.

### BRIEF DESCRIPTION OF DRAWINGS

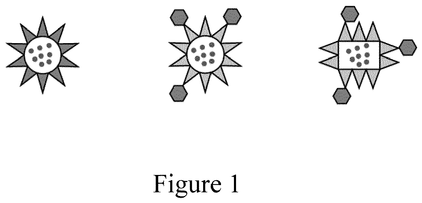

FIG. 1 shows a schematic diagram of an example of the multimer-based antigen complex of the present invention, in which several different forms of the antigen complex are shown. Left: a multimer directly composed of target antigens; middle: a natural multimer backbone with target antigens loaded thereon; right: an artificially constructed multimer backbone with target antigens loaded thereon. The red dot in the figure refers to the immunostimulant in the antigen complex.

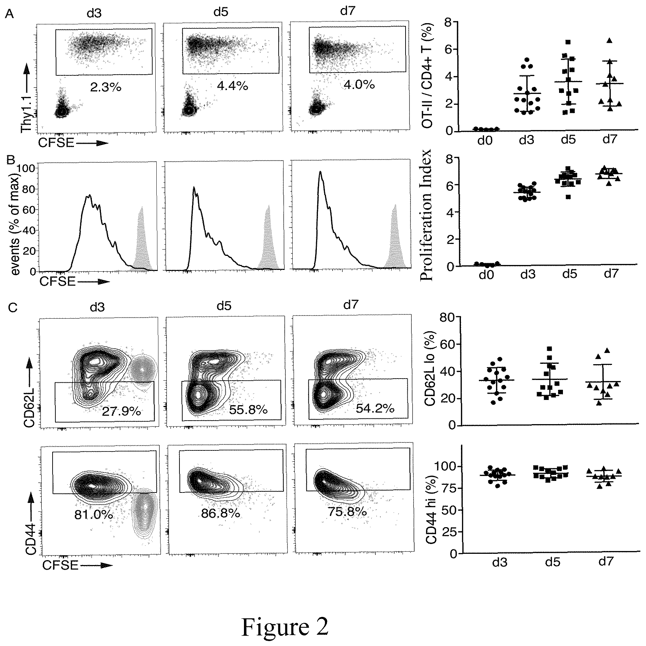

FIG. 2A to FIG. 2C show strong activation of CD4+ T cells upon immunization with multimer Qβ-VLP. After immunizing wild-type mice with Qβ-Qva, CFSE-labeled naive OT-II CD4+ T cells were transferred into the mice. Spleens were harvested from immunized mice at day 3 (d3), day 5 (d5), and day 7 (d7) post-immunization, and from unimmunized mice (d0) as controls. Representative flow cytometry plots and summary data are shown. FIG. 2A shows total CD4+ T cells with gating of Thy1.1+ OT-II cells. FIG. 2B shows a histogram of the fluorescence density of CFSE in Thy1.1+, which is quantified as proliferation index. The gray part in FIG. 2B shows data from unimmunized mice. FIG. 2C shows graphs of up-regulation of T cell activation markers CD44 and down-regulation of CD62L (CD44 hi and CD62 lo). Mean ± SD is shown.

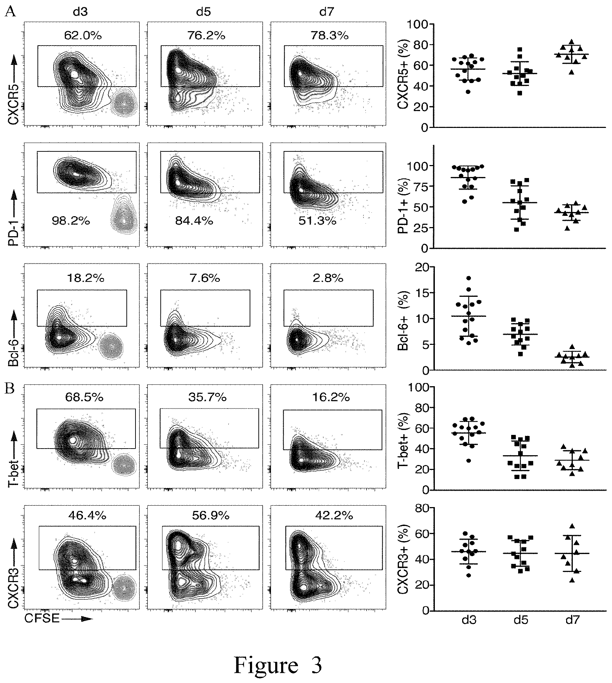

FIG. 3A and FIG. 3B show promotion of differentiation of CD4+ T cells into Tfh cells and Th1 cells upon immunization with multimer Qβ-VLP. After immunizing wild-type mice with Qβ-Qva, CFSE-labeled naive OT-II CD4+ T cells were transferred into the mice. Spleens were harvested from immunized mice at day 3 (d3), day 5 (d5), and day 7 (d7) post-immunization, and from unimmunized mice (d0) as a control. Representative flow cytometry plots and summary data are shown. Thy1.1+ cells are gated for the indicated differentiation markers based on the expression levels of differentiation markers in CFSE-undiluted cells from unimmunized mice. The expression levels of differentiation markers in CFSE-undiluted cells from unimmunized mice are shown in grey part in d3 plots. FIG. 3A shows a significant increase in the proportion of CD4+ T cells positive for Tfh cell specific markers PD1, CXCR5 and Bcl-6, indicating that a large number of CD4+ T cells differentiate into Tfh. FIG. 3B shows a significant increase in the proportion of CD4+ T cells positive for Th1 cell specific markers T-bet and CXCR3, indicating that a large number of CD4+ T cells differentiate into Th1 cells.

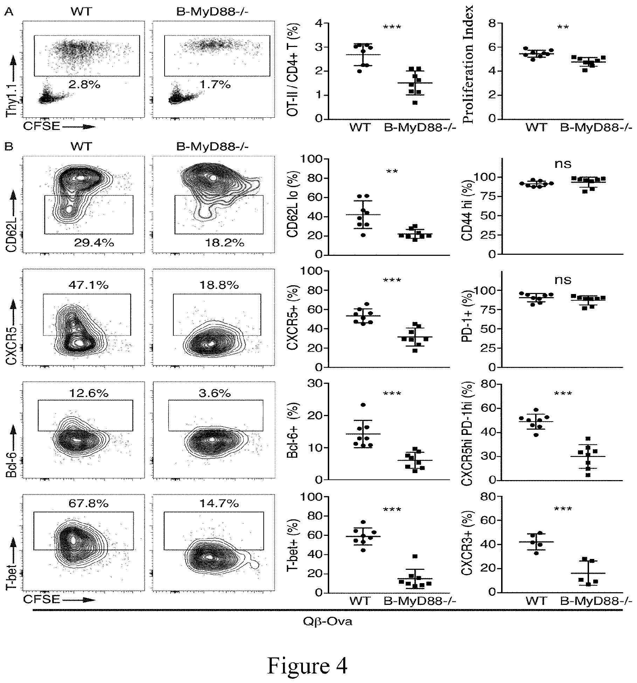

FIG. 4A and FIG. 4B show that MyD88 in B cells was required for activation and differentiation of CD4+ T cells upon immunization with multimer Qβ-VLP. After immunizing wild-type mice or B-MyD88-/- mice with Qβ-Qva, CFSE-labeled naive OT-II CD4+ T cells were transferred into the mice. Spleens were harvested from the mice at day 3 (d3) post-immunization. Representative flow cytometry plots and summary data are shown. FIG. 4A: total CD4+ T cells with gating of Thy1.1+ OT-II cells. FIG. 4B: Thy1.1+ cells from FIG. 4A are gated for the differentiation markers based on the expression levels of differentiation markers in naive CD4+ T cells derived from recipient mice (not shown). Mean ± SD is shown. Unpaired Student's t-test was performed for data analysis. ns: non-significant; ** p<0.01; *** p<0.001.

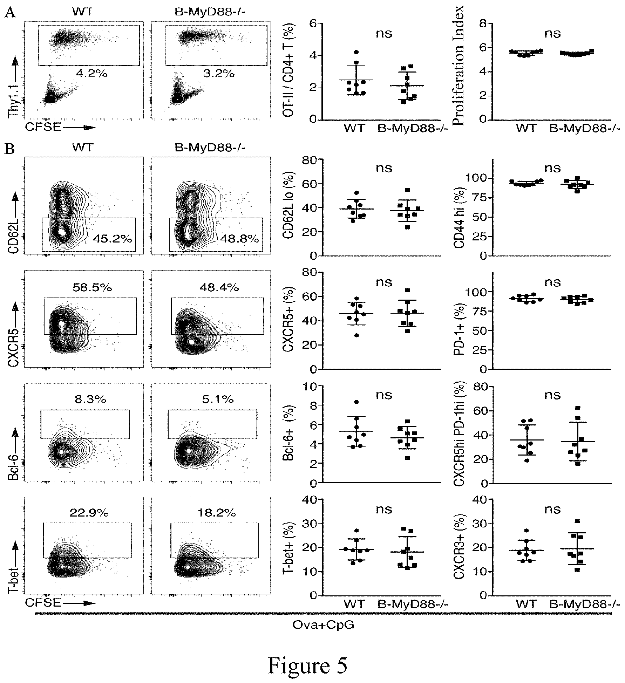

FIG. 5A and FIG. 5B show that MyD88 in B cells was not required for activation and differentiation of CD4+ T cells upon immunization with soluble antigen Ova+CpG. After immunizing wild-type mice or B-MyD88-/- mice with Ova+CpG, CFSE-labeled naive OT-II CD4+ T cells were transferred into the mice. Spleens were harvested from the mice at day 3 (d3) post-immunization. Representative flow cytometry plots and summary data are shown. FIG. 5A: total CD4+ T cells with gating of Thy1.1+ OT-II cells. FIG. 5B: Thy1.1+ cells from FIG. 5A are gated for the differentiation markers based on the expression levels of differentiation markers in naive CD4+ T cells derived from recipient mice. Mean ± SD is shown. Unpaired Student's t-test was performed for data analysis. ns: non-significant.

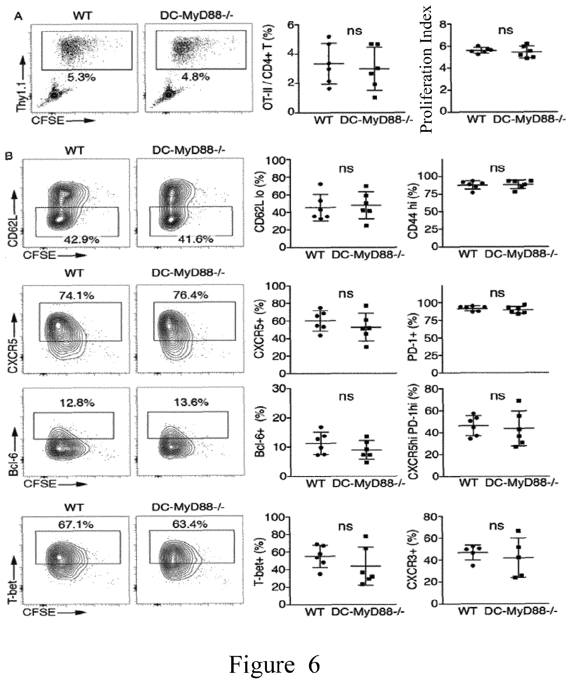

FIG. 6A and FIG. 6B show that MyD88 in DC cells was not required for activation and differentiation of CD4+ T cells upon immunization with multimer Qβ-VLP. After immunizing wild-type mice or DC-MyD88-/- mice with Qβ-Qva, CFSE-labeled naive OT-II CD4+ T cells were transferred into the mice. Spleens were harvested from the mice at day 3 (d3) post-immunization. FIG. 6A: total CD4+ T cells with gating of Thy1.1+ OT-II cells. FIG. 6B: Thy1.1+ OT-II CD4+ T cells are gated for the differentiation markers. Mean ± SD is shown. Unpaired Student's t-test was performed for data analysis. ns: non-significant.

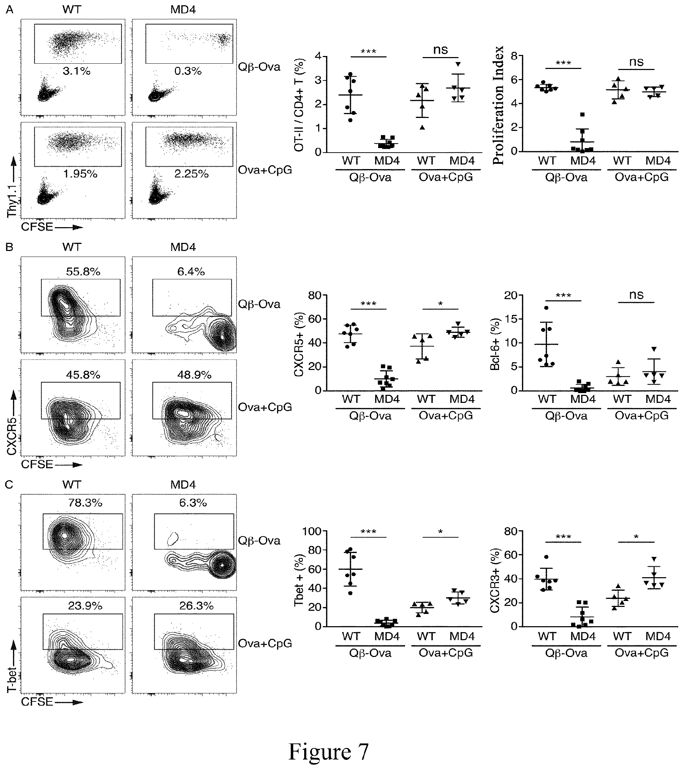

FIG. 7A to FIG. 7C show that mice lacking Qβ-specific B cells failed to induce CD4+ T cell responses upon immunization with multimer Qβ-VLP. After immunizing wild-type mice and MD4 mice with Qβ-Qva or Ova mixed with CpG ODN, CFSE-labeled naive OT-II CD4+ T cells were transferred into the mice. Representative flow cytometry plots and summary data are shown. FIG. 7A: total CD4+ T cells with gating of Thy1.1+ OT-II cells. FIG. 7B and FIG. 7C: Thy1.1+ cells from FIG. 7A are gated for the differentiation markers. Mean ± SD is shown. Unpaired Student's t-test was performed for data analysis. ns: non-significant; ** p<0.01; *** p<0.001.

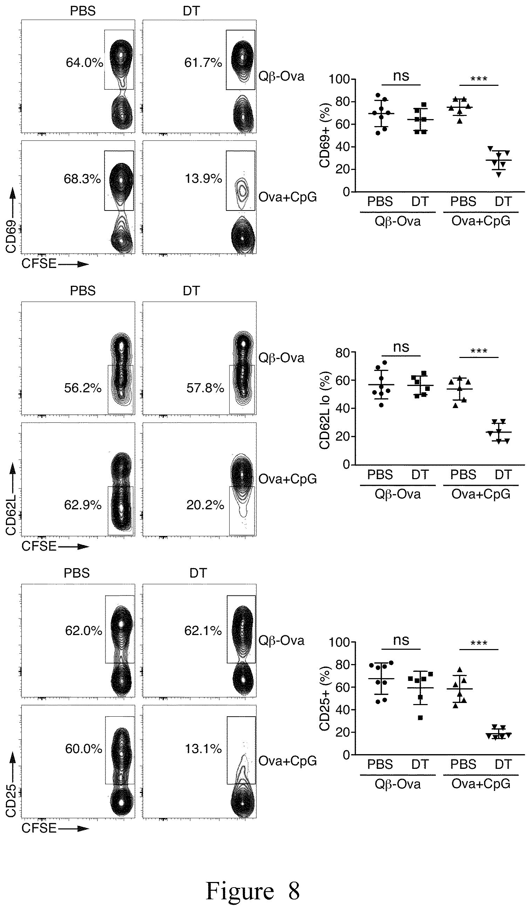

FIG. 8 shows that DCs were not required for CD4+ T cell activation induced by multimer Qβ-VLP. Mice were lethally irradiated to remove immune cells, and reconstituted using BM cells from CD11c-DTR/GFP mice. The mice were treated with PBS or DT during transfer of OT-II CD4+ T cells. After immunizing mice with Qβ-Qva or Ova mixed with CpG ODN, CFSE-labeled naive OT-II CD4+ T cells were transferred into the mice. Spleens were harvested at 24 hours post-immunization. Representative flow cytometry plots and summary data of individual mice are shown. Thy1.1+ CD4+ OT-II T cells are gated for the indicated differentiation markers based on the expression levels of differentiation markers in CFSE-undiluted cells from unimmunized mice. Mean ± SD is shown. Unpaired Student's t-test was performed for data analysis. ns: non-significant; *** p<0.001.

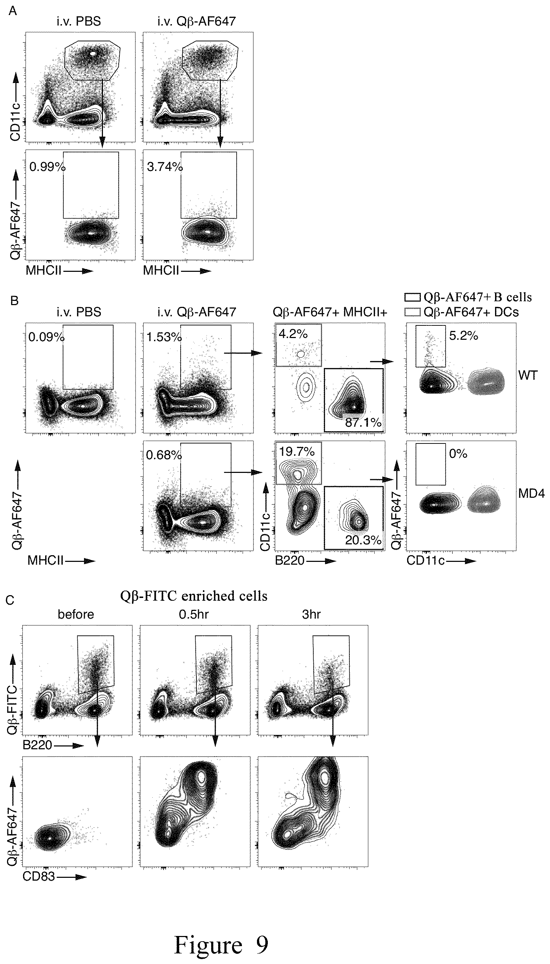

FIG. 9A to FIG. 9C show that Qβ-VLPs were captured by antigen-specific B cells effectively in vivo. FIG. 9A and FIG. 9B: wild-type or MD4 mice were injected intravenously with Qβ-AF647 or PBS, and examined 3 hours later. FIG. 9A: CD11c+ MHCII+ DCs are first gated from total splenocytes, which are further gated for Qβ-AF647+. FIG. 9B: Qβ-AF647+ MHCII+ cells are first gated from total splenocytes, which are further gated as B220+ B cells and CD11c+ DCs. Binding of DCs and B cells to Qβ-AF647 is shown. FIG. 9C: Wild-type mice were injected intravenously with Qβ-AF64, and examined at 0.5 hours and 3 hours after injection. Mice without injection were also examined as controls. Total splenocytes were enriched with Qβ-FITC and anti-FITC magnetic beads. Qβ-FITC+ B220+ B cells are gated from the enriched fraction, which are further displayed for Qβ-AF647 and CD83. Data are representative of at least three independent experiments.

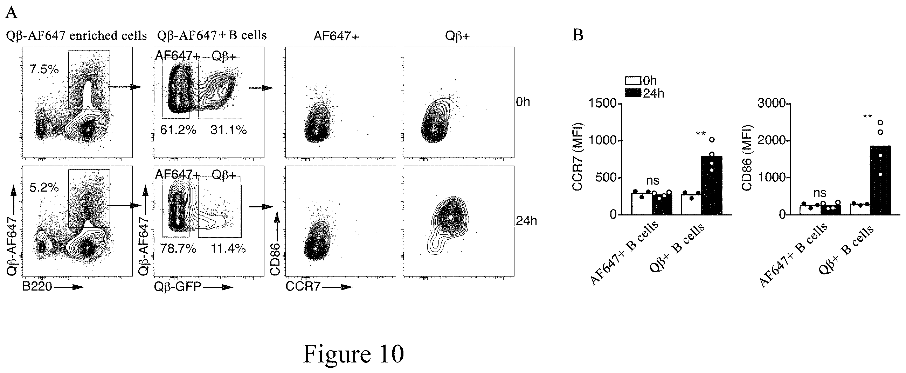

FIG. 10A and FIG. 10B: Wild-type mice were injected intraperitoneally with unlabeled Qβ-VLP. Spleens were harvested 24 hours later, and spleens from unimmunized mice were harvested as controls. Total splenocytes were incubated with Qβ-AF647 and Qβ-GFP, and then enriched with anti-AF647 magnetic beads. FIG. 10A: Qβ-AF647+ B cells are gated from the enriched cell fraction, which are further gated as AF647+ and Qβ+ B cells according to Qβ-GFP. CD86 and CCR7 are shown. FIG. 10B: mean fluorescence intensity (MFI) of CD86 and CCR7 in AF647+ and Qβ+ B cells. Bars represent the mean value. Dots represent the data from individual mice. Unpaired Student's t-test was performed for data analysis. ns: non-significant; ** p<0.01.

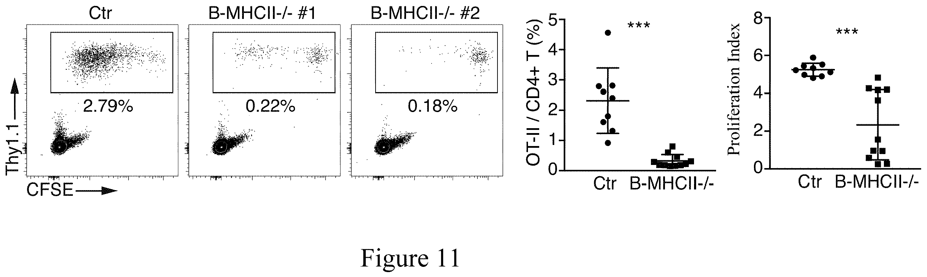

FIG. 11 shows that antigen presentation by B cells was required for CD4+ T cell activation induced by Qβ-VLPs. B-MHCII-/- and control mice were generated by transplanting mixed BM cells from µMT and MHCII-/- (B-MHCII-/-) or WT (control) mice into lethally irradiated mice. After immunizing mice with Qβ-Qva, CFSE-labeled naive OT-II CD4+ T cells were transferred into the mice. Spleens were harvested from the mice at day 3 (d3) post-immunization. Representative flow cytometry plots and summary data are shown. Thy1.1+ OT-II cells are gated from total CD4+ T cells. For B-MHCII-/-, two different representative plots are shown, with #1 exhibiting low level of CFSE dilution. Mean ± SD is shown. Unpaired Student's t-test was performed for data analysis. ns: non-significant; * p<0.05; ** p<0.01; *** p<0.001.

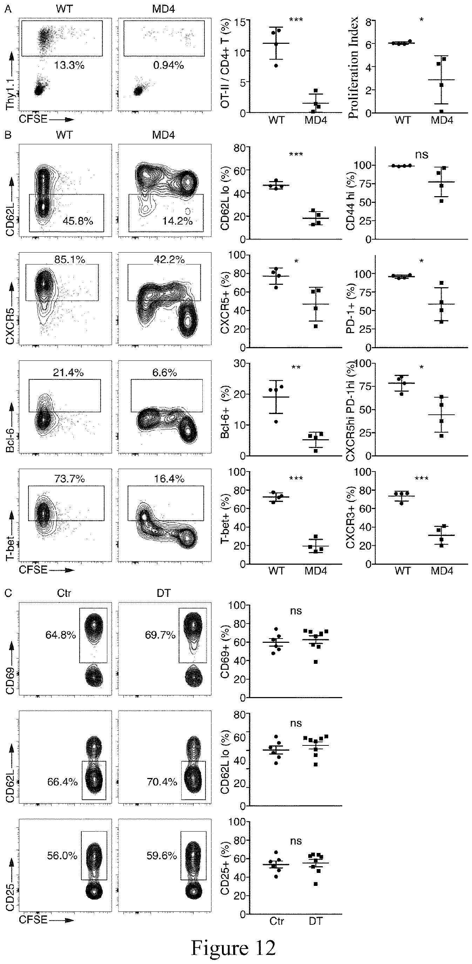

FIG. 12A to FIG. 12C show that antigen-specific B cells were involved in the CD4+ T cell response induced by influenza viruses. CFSE-labeled naive OT-II CD4+ T cells were transferred into WT and MD4 mice (FIG. 12A and FIG. 12B), or CD11c-DTR/GFP BM chimeric mice treated with PBS (control) or DT (C), followed by immunization with PR8-Ova. Spleens were harvested at day 3 (FIG. 12A and FIG. 12B) or 24 hours (FIG. 12C) post-immunization. Representative flow cytometry plots and summary data of individual mice are shown. FIG. 12A: total CD4+ T cells with gating of Thy1.1+ OT-II cells. FIG. 12B and FIG. 12C: Thy1.1+ CD4+ T cells are gated for the indicated markers. Mean ± SD is shown. Unpaired Student's t-test was performed for data analysis. ns: non-significant; * p<0.05; ** p<0.01; *** p<0.001.

### DETAILED DESCRIPTION

### Multimer-based antigen complex

In an aspect, the present disclosure relates to a multimer-based antigen complex.

In some embodiments, the multimer-based antigen complex comprises:
i) a multimer assembled from a plurality of subunits; and
ii) an immunostimulant,
wherein the plurality of subunits comprise or consist of a target antigen, and wherein the immunostimulant is packaged in the multimer, or attached to the multimer by physical adsorption or chemical linkage.

In other embodiments, the multimer-based antigen complex comprises:
i) a multimer assembled from a plurality of subunits;
ii) a loaded target antigen; and
iii) an immunostimulant,
wherein the target antigen is attached to the surface of the multimer by physical adsorption or chemical linkage, or fused with at least a part of the plurality of subunits by gene fusion, which fusion does not affect the assembly of the multimer, and the target antigen is displayed on the surface of the multimer after the multimer is assembled; and wherein the immunostimulant is packaged in the multimer, or attached to the multimer by physical adsorption or chemical linkage.

FIG. 1 shows a schematic diagram of an example of the multimer-based antigen complex of the present disclosure, in which several different forms of the antigen complex are shown.

When referring to the "multimer-based antigen complex", the above term also encompasses the case of a mixture of a plurality of multimer-based antigen complexes. For example, the term "multimer-based antigen complex" may mean a mixture of two or more multimer-based antigen complexes. In some embodiments, the two or more multimer-based antigen complexes may each have components i) and ii), or components i), ii) and iii) described above. In other words, where the "multimer-based antigen complex" means two or more multimer-based antigen complexes, some of the antigen complexes may have components i) and ii) described above, while other antigen complexes may have components i), ii) and iii) described above.

The multimer-based antigen complexes of the present disclosure may be used to activate B cells, or further activate CD4+ T cells through the recognition and presentation by B cells, and promote differentiation of CD4+ T cells into Tfh and Th1.

In an organism, most cells do not express MHC II. Cells that are capable of expressing MHC II and have antigen-mediated specific bind to CD4+ T cells are referred to as antigen presenting cells (APCs). Dendritic cells (DCs), B cells and macrophages are the main types of antigen presenting cells. Although these types of cells all express MHC II and can stimulate activation of CD4+ T cells under certain conditions, it is generally believed that only DCs are capable of activating CD4+ T cells in the initial state by antigen presentation (through MHC class II molecules) and generation of cytokines. CD4 T cells in the initial state are also referred to as naive CD4+ T cells. Whether they can be effectively activated determines the strength of the subsequent immune response, which step is an important target for various measures to enhance the immune response. Due to the critical role of DCs in this step, DCs are currently regarded as a key target in the research and development of various vaccines.

In this application, the inventors of the present disclosure have surprisingly discovered that although most antigens activate CD4+ T cells through DCs, a special form of antigens, i.e., the multimer-based antigen complex, can perform initial activation to naive CD4+ T cells through B cells. That is, it is able to induce activation and differentiation of CD4+ T cells through antigen presentation in the absence of DCs.

Such ability of B cells is closely related to the immunoglobulin receptor (B cell receptor, BCR) expressed therefrom and the innate immune signaling pathway. BCR is actually immunoglobulin in form of transmembrane, which is generated as a result of a DNA-level gene rearrangement process at specific BCR gene-related sites during the development of B cells, i.e. V(D)J rearrangement. This lymphocyte-specific V(D)J rearrangement process allows different B cells to express different BCRs, with up to 10¹²-10¹⁵ types of BCRs possibly generated. Due to the abundance of BCR, B cells utilize a BCR-expressing population of cells with higher affinity for antigens in B cell population upon recognition of antigens. After being exposed to the antigens, these cells can be activated and further differentiate into immune effector cells. It is found in our research that when these cells are activated by the multimer-based antigen complex described herein, their ability as APCs is greatly enhanced and can completely replace the role of DCs.

In some embodiments, the multimer may have a diameter of about 10 nm to about 1000 nm, for example, about 10 nm to about 500 nm, about 10 nm to about 300 nm, about 10 nm to about 200 nm, about 10 nm to about 100 nm, about 10 nm to about 50 nm, about 20 nm to about 1000 nm, about 20 nm to about 500 nm, about 20 nm to about 300 nm, about 20 nm to about 200 nm, about 20 nm to about 100 nm, about 20 nm to about 50 nm, about 50 nm to about 1000 nm, about 50 nm to about 500 nm, about 50 nm to about 300 nm, about 50 nm to about 200 nm, or about 50 nm to about 100 nm.

In some embodiments, the multimer may comprise at least 4 subunits, for example at least 10 subunits, at least 20 subunits, at least 50 subunits, at least 100 subunits, or at least 200 subunits. The multimer may have 10 to 1000 subunits, for example 20 to 500 subunits, 50 to 300 subunits, or 100 to 200 subunits.

In any embodiment of the multimer-based antigen complex described above, the immunostimulant may comprise a bacteria-derived ssRNA, an artificially synthesized ssRNA or a derivative thereof, an artificially synthesized CpG-containing oligonucleotide, an interferon, a cytokine, or any combination thereof. In some embodiments, the bacteria-derived ssRNA may be an E. coli-derived ssRNA. In some embodiments, the interferon may be selected from type I interferon, type II interferon, type III interferon, and a combination thereof. In some embodiments, the cytokine may be selected from IL-6, IL-12, IL21, and a combination thereof.

In the process of activation of lymphocytes such as B cells and T cells, two factors are usually required: one is an antigen receptor, i.e., stimulation and signaling of BCRs and TCRs; the other is stimulation of immune signals or cytokines. These two stimuli together determine whether lymphocytes can be activated and the direction of functional differentiation after activation. The immunostimulant herein refers to a substance that can implement the second stimulation, mainly comprising two types: ligand stimulants for innate immune receptors and pro-inflammatory cytokines.

Examples of the types of immunostimulants that can be used in the antigen composition of the present application are listed in Table 1 below.

**Table 1: Immunostimulant**

| Innate immune receptor stimulant | Toll-like receptor ligand stimulant | TLR1 ligand stimulant |
|---|---|---|
| | | TLR2 ligand stimulant |
| | | TLR3 ligand stimulant |
| | | TLR4 ligand stimulant |
| | | TLR5 ligand stimulator |
| | | TLR6 ligand stimulant |
| | | TLR7 ligand stimulant |
| | | TLR8 ligand stimulator |
| | | TLR9 ligand stimulant |
| | | TLR10 ligand stimulant |
| | | TLR11 ligand stimulant |
| | | TLR12 ligand stimulant |
| | | TLR13 ligand stimulant |
| | NOD-like receptor ligand stimulant | |
| | RIG-I-like receptor ligand stimulant | |
| Cytokines | Interleukin | IL-6 |
| | | IL-12 |
| | | IL-21 |
| | | IL-4 |
| | Interferon | IFN-a |
| | | IFN-b |
| | | IFN-g |
| | Tumor necrosis factor family | TNF |
| | | CD70 |
| | | TNFSF8 |
| | | TNFSF13 |
| | | TNFSF13B |

Specific examples of Toll-like receptor ligand stimulants are listed in Table 2 below.

**Table 2: Toll-like receptor ligand stimulant**

| Receptor | Ligand |
|---|---|
| TLR 1 | Various tricyclic lipopeptides |
| TLR 2 | Various glycolipids |
| | Various lipopeptides |
| | Various lipoproteins |
| | Lipoteichoic acid |
| | HSP70 |
| | Zymosan (Beta-glucan) |
| | Various other ligands |
| TLR 3 | Double-stranded RNA, poly I:C |
| TLR 4 | Lipopolysaccharide |
| | Several heat shock proteins |
| | Fibrinogen |
| | Heparan sulfate fragment |
| | Hyaluronic acid fragment |
| | Nickel |
| | Various opioids |
| TLR 5 | Bacterial flagellin |
| | Profilin |
| TLR 6 | Various diacyl peptides |
| TLR 7 | Imidazoquinoline |
| | Loxoribine (a guanosine analogue) |
| | Bropirimine |
| | Resiquimod |
| | Single-stranded RNA |
| TLR 8 | Small synthetic compounds; single-stranded viral RNA, phagocytic bacterial RNA |
| TLR 9 | Unmethylated CpG oligodeoxynucleotide DNA |
| TLR 10 | Triacylated lipopeptide |
| TLR 11 | Profilin |
| TLR 12 | Profilin |
| TLR 13 | Bacterial ribosomal RNA sequence "CGGAAAGACC" |

The multimer-based antigen complex of the present disclosure may comprise one immunostimulant, or two or more different immunostimulants.

In addition, in addition to the immunostimulants specifically exemplified above, various natural and artificial immunostimulants for promoting immune responses are known in the art, and can be selected for constructing the antigen complex of the present disclosure. The immunostimulant and the multimer may be combined without particular limitation, for example, packaged in the multimer, or attached to the surface of the multimer by physical adsorption or chemical linkage to exert their immunostimulatory function. In the case where the immunostimulant is packaged in the multimer, the immunostimulant can be introduced during assembly of the multimer, such that the subunits of the multimer pack the immunostimulant inside the multimer during the assembly.

In a further embodiment of the multimer-based antigen complex described above, the multimer may be a virus-like particle, another natural multimer or an artificially synthesized multimer.

The virus-like particles are biologics that are similar in structure with viruses, but do not contain viral genetic materials. The virus-like particle usually consists of multiple copies of one or more proteins, with diameter varies from tens of nanometers to thousands of nanometers. The surface of virus-like particle presents repeatedly arranged antigen epitopes, which greatly enhances its ability to activate B cells. The virus-like particle can contain natural or artificial nucleic acid substances inside, and other compounds can also be artificially added as immune stimulants. The immune stimulant is important for the immune response induced by the virus-like particle, especially for the B cell response.

In addition to the virus-like particle, some naturally-occurring polysaccharide compounds are also natural multimers, and can be further formed into particle-like structure on such basis. The multimer can also be used in the antigen complex of the present disclosure to activate B cells, and further activate CD4+ T cells through recognition and presentation by B cells, and promote differentiation of CD4+ T cells into Tfh and Th1. In addition, some artificially involved and engineered proteins can also form multimers. Non-protein substances can also form multivalent particle preparation, such as artificially synthetic nanoparticles. After modification of the surfaces of these artificially synthesized multimers with specific chemical groups, the target antigen can be loaded on the multimers through physical adsorption or chemical linkage.

The multimer may be assembled from multiple copies of one subunit, or may be assembled from multiple copies of two or more subunits. There is no specific restriction on selection of multimers, and those skilled in the art may select various multimer structures known in the art for constructing the antigen complex of the present disclosure.

In some embodiments, the virus-like particle may comprise or consist of Qβ protein, HBcAg or AP205.

Bacterial phage Qβ is an icosahedral RNA virus with a diameter of 30 nm. Its host is Escherichia coli. Qβ enters its host cell by binding to F fimbriae on the surface of the bacteria. The first 133 amino acids of the phage Qβ capsid protein can be expressed in other cells such as E. coli or yeast by plasmid transformation, and self-assembled into particles with a diameter of 30 nm. The self-assembly process of the phage Qβ capsid protein does not require its own genetic material or the assistance of other proteins. The assembled particles are not infectious to any cell (including prokaryotic and eukaryotic cells).

HBcAg (core antigen) is a hepatitis B virus protein, which is an antigen present on the surface of the nucleocapsid core (the innermost layer of the hepatitis B virus). In cells infected by the hepatitis B virus, HBcAg is related to packaging of the viral nucleic acid. Although the hepatitis B virus only infects eukaryotic cells, HBcAg may be expressed in other cells such as E. coli or yeast through plasmid transformation, and self-assembled into particles with a diameter of about 30 nm. The self-assembly process of the HBcAg does not require its own genetic material or the assistance of other proteins. The assembled particles are not infectious to any cell (including prokaryotic and eukaryotic cells).

Bacterial phage AP205 is an icosahedral RNA virus with a diameter of 30 nm. Its host is Acinetobacter. AP205 capsid protein may be expressed in other cells such as E. coli or yeast through plasmid transformation, and self-assembled into particles with a diameter of 30 nm. The self-assembly process of the AP205 capsid protein does not require its own genetic material or the assistance of other proteins. The assembled particles are not infectious to any cell (including prokaryotic and eukaryotic cells).

In some embodiments, Qβ protein may have the amino acid sequence shown below:

MAKLETVTLGNIGKDGKQTLVLNPRGVNPTNGVASLSQAGAVPALEKRV
TVSVSQPSRNRKNYKVQVKIQNPTACTANGSCDPSVTRQAYADVTFSFTQ
YSTDEERAFVRTELAALLASPLLIDAIDQLNPAY (SEQ ID NO: 1)

In some embodiments, HBcAg may have the amino acid sequence shown below:

MDIDPYKEFGATVELLSFLPSDFFPSVRDLLDTASALYREALESPEHCSPH
HTALRQAILCWGELMTLATWVGNNLEDPASRDLVVNYVNTNMGLKIRQ
LLWFHISCLTFGRETVLEYLVSFGVWIRTPPAYRPPNAPILSTLPETTVVRR
RDRGRSPRRRTPSPRRRRSQSPRRRRSQSRESQC (SEQ ID NO: 2)

In some embodiments, AP205 may have the amino acid sequence shown below:

MANKPMQPITSTANKIVWSDPTRLSTTFSASLLRQRVKVGIAELNNVSGQ
YVSVYKRPAPKPEGCADACVIMPNENQSIRTVISGSAENLATLKAEWETH
KRNVDTLFASGNAGLGFLDPTAAIVSSDTTA (SEQ ID NO: 3)

In some embodiments, the multimer, such as virus-like particle, comprises or consists of the target antigen. That is, at least one subunit of the multimer itself serves as the target antigen. For example, in the case where the multimer is a virus-like particle, the subunit as the target antigen may be Qβ protein, HBcAg or AP205, or other virus-derived proteins. The multimer such as virus-like particle may contain multiple copies of only one subunit, or may contain two or more subunits. In the case where the multimer such as virus-like particle contains multiple copies of one subunit, the subunit itself can serve as the target antigen. In the case where the multimer contains two or more subunits, at least one of the subunits may serve as the target antigen.

In other embodiments, the antigen complex comprises a loaded target antigen. The loaded target antigen is not particularly limited, and may be any protein, polypeptide, nucleic acid or small molecule that is immunogenic and can be specifically recognized by components of the immune system. Various target antigens for immunization are known in the art, and those skilled in the art may select a specific target antigen as needed to construct the antigen complex of the present disclosure.

In addition, the method for loading the target antigen is not particularly limited, and method such as physical adsorption, chemical ligation, and gene fusion may be employed. When the target antigen is loaded by means of physical adsorption or chemical linkage, the timing of loading is not particularly limited. For example, in some embodiments, the subunits of the multimer may be contacted with the target antigen (for example, physical adsorption or chemical action occurs), and then assembled into a multimer, such that the target antigen is loaded on the surface of the multimer. In this case, the binding of the multimer subunits to the target antigen does not affect their assembly into the multimer. In other embodiments, the target antigen may be introduced after assembly of subunits into the multimer, such that the target antigen is attached to the surface of the multimer by physical adsorption or chemical linkage.

When the target antigen is added by means of gene fusion, that is, the nucleotide sequence encoding the target antigen is fused with the nucleotide sequence encoding the subunits of the multimer through genetic recombination technology, and expressed as a fusion protein, the target antigen may be fused to only part of the subunits constituting the multimer. In other words, in the case where the multimer contains multiple copies of only one subunit, the target antigen may be fused with all or only a part of the multiple copies. In the case where the multimer contains two or more subunits, the target antigen may be fused to at least one of the two or more subunits. In addition, the target antigen may be fused to all or only a part of the at least one subunit.

The methods for constructing a fusion protein by genetic recombination technology are well known in the art. In addition, those skilled in the art may choose a suitable fusion method according to various conditions such as the type, size and immunogenicity of the target antigen used, and copy number of the subunits of the multimer, such that after fusion, the target antigen does not affect assembly of the multimer.

In some embodiments, the loaded target antigen may be a bacteria- or virus-derived antigen. The specific types of the bacteria- or virus-derived antigens are not particularly limited, and exemplary antigens such as those listed in Table 3 can be used.

**Table 3: Bacteria- or virus-derived antigen**

| Mycobacterium tuberculosis | DosR |
|---|---|
| | Ag85 |
| | ESAT6 |
| | Crystalline |
| | CFP10 |
| | Rv2031c |
| Epstein-Barr virus | EBNA-1 |
| | EBNA-2 |
| | EBNA-3A |
| | EBNA-3B |
| | EBNA-LP |
| | LMP-1 |
| | LMP-2A |
| | LMP-2B |
| | EBER |
| | Gp350 |
| Hepatitis B virus | HBsAg |
| | Pre-S 1 |
| Plasmodium | CSP |
| | MSP1 |
| | MSP3 |
| | DBP |

In some embodiments, the bacteria- or virus-derived antigen may be a mycobacterium tuberculosis-derived antigen, for example, selected from crystallin and Rv3133c.

In other embodiments, the bacteria- or virus-derived antigen may be a superbacteria-derived antigen, for example, selected from Klebsiella pneumoniae carbapenemase and penicillin binding protein.

In still other embodiments, the bacteria- or virus-derived antigen may be a lentivirus-derived antigen, for example, selected from HBV pre-S1 antigen and EBV LMP1 antigen.

In other embodiments of the multimer-based antigen complex described above, the antigen complex comprises a loaded target antigen, which may be a tumor-associated antigen.

The tumor-associated antigen used is not particularly limited, and may be any antigen associated with tumor development or aggressiveness. The term "tumor-associated antigen" refers to an antigen that is differentially expressed by cancer cells and can therefore be exploited to target cancer cells. The tumor-associated antigen is an antigen that can potentially stimulate significant tumor-specific immune responses. Some of these antigens are encoded , though not necessarily expressed, by normal cells. These antigens can be characterized as those that are normally silent (i.e., not expressed) in normal cells, those that are expressed only at certain stages of differentiation, and those that are temporally expressed, such as embryonic and fetal antigens. Other tumor-associated antigens are encoded by mutant cellular genes such as oncogenes (e.g., activated ras oncogene), suppressor genes (e.g., mutant p53), and fusion proteins resulting from internal deletions or chromosomal translocations. Other tumor-associated antigens can be encoded by viral genes, such as those carried by RNA and DNA tumor viruses.

In some embodiments, the intact cancer antigen is used, while in other embodiments, a peptide epitope of the cancer antigen (prepared either by proteolytic digestion or recombinantly) are used. Therefore, non-limiting examples of tumor or tumor-associated antigen in the multimer-based antigen complex herein include, but are not limited to, Her2, prostate stem cell antigen (PSCA), PSMA (prostate-specific membrane antigen), β-catenin-m, B cell maturation antigen (BCMA), alpha-fetoprotein (AFP), carcinoembryonic antigen (CEA), cancer antigen-125 (CA-125), CA19-9, calretinin, MUC-1, epithelial membrane protein (EMA), epithelial tumor antigen (ETA), tyrosinase, Mammaglobin-A, melanoma-associated antigen (MAGE), CD34, CD45, CD99, CD117, chromogranin, cytokeratin, desmin, glial fibrillary acidic protein (GFAP), gross cystic disease fluid protein (GCDFP-15), EBV, gp100, HMB-45 antigen, protein melan-A (melanoma antigen recognized by T lymphocytes; MART-1), livin, survivin, myo-D1, muscle-specific actin (MSA), neurofilament, neuron-specific enolase (NSE), placental alkaline phosphatase, synaptophysin, thyroglobulin, thyroid transcription factor-1, the dimer form of pyruvate kinase isoenzyme M2 (tumor M2-PK), CD19, CD22, CD27, CD30, CD70, GD2 (ganglioside G2), EphA2, CSPG4, CD138, FAP (fibroblast activation protein), CD171, kappa, lambda, 5T4, αᵥβ₆ integrin, B7-H3, B7-H6, CAIX, CD19, CD20, CD22, CD30, CD33, CD44, CD44v6, CD44v7/8, CD70, CD123, EGFR, EGP2, EGP40, EpCAM, fetal AchR, FRα, GAGE, GD3, HLA-A1+MAGE1, MAGE-3, HLA-A1+NY-ESO-1, IL-11Rα, IL-13Rα2, Lewis-Y, Mucl6, NCAM, NKG2D Ligands, NY-ESO-1, PRAME, ROR1, SSX, Survivin, TAG72, TEMs, VEGFR2, EGFRvIII (epidermal growth factor variant III), sperm protein 17 (Sp17), mesothelin, PAP (prostatic acid phosphatase), prostein, TARP (T cell receptor γ alternate reading frame protein), Trp-p 8, STEAP1 (six-transmembrane epithelial antigen of the prostate 1), HSP70-2/m and HLA-A2-R170J, tyrosinase, abnormal ras protein or abnormal p53 protein.

In some embodiments, the tumor antigen may be selected from Her2, p53, or tumor neoantigen.

### Methods for activating T cells and promoting T cell differentiation

In one aspect, the present disclosure relates to a method for activating CD4+ T cells, comprising the step of contacting B cells with CD4+ T cells, wherein the B cells have been activated using a multimer-based antigen complex or an equivalent method, and the multimer-based antigen complex comprises:
i) a multimer assembled from a plurality of subunits; and
ii) an immunostimulant,
wherein the plurality of subunits comprise or consist of a target antigen, and wherein the immunostimulant is packaged in the multimer, or attached to the multimer by physical adsorption or chemical linkage;
alternatively, the antigen complex comprising:
i) a multimer assembled from a plurality of subunits;
ii) a loaded target antigen; and
iii) an immunostimulant,
wherein the target antigen is attached to the surface of the multimer by physical adsorption or chemical linkage, or fused with at least a part of the plurality of subunits by gene fusion, which fusion does not affect the assembly of the multimer, and the target antigen is displayed on the surface of the multimer after the multimer is assembled; and wherein the immunostimulant is packaged in the multimer, or attached to the multimer by physical adsorption or chemical linkage.

In another aspect, the present disclosure relates to a method for promoting differentiation of CD4+ T cells into Tfh and/or Th1 cells, comprising the step of contacting B cells with CD4+ T cells, wherein the B cells have been activated using a multimer-based antigen complex or an equivalent method, and the multimer-based antigen complex comprises:
i) a multimer assembled from a plurality of subunits; and
ii) an immunostimulant,
wherein the plurality of subunits comprise or consist of a target antigen, and wherein the immunostimulant is packaged in the multimer, or attached to the multimer by physical adsorption or chemical linkage;
alternatively, the antigen complex comprising:
i) a multimer assembled from a plurality of subunits;
ii) a loaded target antigen; and
iii) an immunostimulant,
wherein the target antigen is attached to the surface of the multimer by physical adsorption or chemical linkage, or fused with at least a part of the plurality of subunits by gene fusion, which fusion does not affect the assembly of the multimer, and the target antigen is displayed on the surface of the multimer after the multimer is assembled; and wherein the immunostimulant is packaged in the multimer, or attached to the multimer by physical adsorption or chemical linkage.

In yet another aspect, the present disclosure relates to a method for activating CD4+ T cells, comprising the following steps:
a) contacting a multimer-based antigen complex with a population of B cells,
   the antigen complex comprising:
   i) a multimer assembled from a plurality of subunits; and
   ii) an immunostimulant,
   wherein the plurality of subunits comprise or consist of a target antigen, and wherein the immunostimulant is packaged in the multimer, or attached to the multimer by physical adsorption or chemical linkage;
   alternatively, the antigen complex comprising:
   i) a multimer assembled from a plurality of subunits;
   ii) a loaded target antigen; and
   iii) an immunostimulant,
   wherein the target antigen is attached to the surface of the multimer by physical adsorption or chemical linkage, or fused with at least a part of the plurality of subunits by gene fusion, which fusion does not affect the assembly of the multimer, and the target antigen is displayed on the surface of the multimer after the multimer is assembled; and wherein the immunostimulant is packaged in the multimer, or attached to the multimer by physical adsorption or chemical linkage,
   wherein at least a part of the population of B cells are capable of recognizing at least one of the subunits;
b) incubating the antigen complex with the population of B cells to allow B cells to recognize and process the antigen complex, and present the target antigen on the cell surface; and
c) contacting the population of B cells with CD4+ T cells to activate the CD4+ T cells.

In another aspect, the present disclosure relates to a method for promoting differentiation of CD4+ T cells into Tfh and/or Th1 cells, comprising the following steps:
a) contacting a multimer-based antigen complex with a population of B cells,
   the antigen complex comprising:
   i) a multimer assembled from a plurality of subunits; and
   ii) an immunostimulant,
   wherein the plurality of subunits comprise or consist of a target antigen, and wherein the immunostimulant is packaged in the multimer, or attached to the multimer by physical adsorption or chemical linkage;
   alternatively, the antigen complex comprising:
   i) a multimer assembled from a plurality of subunits;
   ii) a loaded target antigen; and
   iii) an immunostimulant,
   wherein the target antigen is attached to the surface of the multimer by physical adsorption or chemical linkage, or fused with at least a part of the plurality of subunits by gene fusion, which fusion does not affect the assembly of the multimer, and the target antigen is displayed on the surface of the multimer after the multimer is assembled; and wherein the immunostimulant is packaged in the multimer, or attached to the multimer by physical adsorption or chemical linkage,
   wherein at least a part of the population of B cells are capable of recognizing at least one of the subunits;
b) incubating the antigen complex with the population of B cells to allow B cells to recognize and process the antigen complex, and present the target antigen on the cell surface; and
c) contacting the B cells with CD4+ T cells, such that the CD4+ T cells are differentiated into Tfh and/or Th1.

T cells or T lymphocytes are a type of lymphocytes that play a central role in cell-mediated immunity. They can be distinguished from other lymphocytes, such as B cells and natural killer cells (NK cells), by the presence of a T-cell receptor (TCR) on the cell surface.

CD4+ T cells, also referred as helper T helper cells (Th cells), assist other white blood cells in immunological processes, including maturation of B cells into plasma cells and memory B cells, as well as activation of cytotoxic T cells and macrophages. Th cells express CD4 on their surface. Th cells become activated when they are presented with peptide antigens by MHC class II molecules on the surface of antigen presenting cells (APCs). These cells can differentiate into one of several subtypes, including Th1, Th2, Th3, Th17, Th9 or Tfh, which secrete different cytokines to facilitate different types of immune responses.

Tfhs, i.e., follicular helper T cells, are an important subset of CD4+ T cells, which are mainly involved in the process of germinal center reaction. Tfhs are essential for formation and maintenance of a germinal center. By inducing and maintaining the effects of germinal center B cells, Tfhs may promote various effects such as antibody production, conversion of antibody types, antibody affinity maturation, neutralizing antibody production, and increase in the breadth of the antibody profile. Therefore, how to efficiently produce Tfh cells is a key link in the research and development of a variety of vaccines.

Th1s, i.e., Type 1 helper T cells, are another important subset of CD4+ T cells, which play a key role in antiviral and antibacterial responses, especially those against an intracellular bacterial infection, such as a tuberculosis infection. In addition, CD4+ T cells also play an important role in anti-tumor immunity.

In some embodiments of the methods described above, the population of B cells is a population of B cells present in the subject. For example, the population of B cells may be a population of B cells naturally occurring in the subject, or a population of B cells transferred to the subject. In this case, the method for activating CD4+ T cells and the method for promoting differentiation of CD4+ T cells of the present disclosure can occur in the subject. That is, by directly administering the multimer-based antigen complexes of the present disclosure to the subject, they can be recognized by the population of B cells in the subject, thereby activating CD4+ T cells and promoting differentiation of the CD4+ T cells into Tfh and/or Th1.

In other embodiments of the methods described above, the population of B cells may be a population of B cells isolated from peripheral blood or a lymphoid organ, such as thymus, spleen, and tonsils, of a donor.

In a further embodiment of the methods described above, after step b), the method may further comprise the step of screening, enriching and/or amplifying B cells that recognize the subunit. In other embodiments, after step b), the method may further comprise a step of screening the B cells that recognize the subunit, and introducing a gene sequence encoding an immunoglobulin receptor into the population of B cells, to increase the number of B cells that recognize the subunit in the population.

The ability of B cells to specifically recognize an antigen comes from immunoglobulin receptors (B cell receptors, BCRs) expressed on their surface. Different B cells can express different BCRs, and up to 10¹²-10¹⁵ types of BCRs may be produced in an individual. Through the step of screening, enriching and/or amplifying the B cells that recognize the subunit, or introducing the gene sequence encoding the BCR that recognizes the subunit into the population of B cells, the number of B cells that recognize the subunit can be increased, thereby increasing the efficiency of activating CD4+ T cells and/or promoting differentiation of CD4+ T cells.

In any embodiment of the methods described above, the multimer may have a diameter of about 10 nm to about 1000 nm, for example, about 10 nm to about 500 nm, about 10 nm to about 300 nm, about 10 nm to about 200 nm, about 10 nm to about 100 nm, about 10 nm to about 50 nm, about 20 nm to about 1000 nm, about 20 nm to about 500 nm, about 20 nm to about 300 nm, about 20 nm to about 200 nm, about 20 nm to about 100 nm, about 20 nm to about 50 nm, about 50 nm to about 1000 nm, about 50 nm to about 500 nm, about 50 nm to about 300 nm, about 50 nm to about 200 nm, or about 50 nm to about 100 nm.

In any embodiment of the methods described above, the multimer may comprise at least 4 subunits, for example at least 10 subunits, at least 20 subunits, at least 50 subunits, at least 100 subunits, or at least 200 subunits. The multimer may have 10 to 1000 subunits, for example 20 to 500 subunits, 50 to 300 subunits, or 100 to 200 subunits.

In any embodiment of the methods described above, the immunostimulant may comprise a bacteria-derived ssRNA, an artificially synthesized ssRNA or a derivative thereof, an artificially synthesized CpG-containing oligonucleotide, an interferon, a cytokine, or any combination thereof. In some embodiments, the bacteria-derived ssRNA may be an E. coli-derived ssRNA. In some embodiments, the interferon may be selected from type I interferon, type II interferon, type III interferon, and a combination thereof. In some embodiments, the cytokine may be selected from IL-6, IL-12, IL21, and a combination thereof.

In some embodiments, one or more selected from the immunostimulants listed in Table 1 and Table 2 above, or other natural or artificial immunostimulants known in the art may be used.

In a further embodiment of the methods described above, the multimer may be a virus-like particle, another natural multimer or artificially synthesized multimer. The multimer may be assembled from multiple copies of one subunit, or may be assembled from multiple copies of two or more subunits. Selection of the multimer is not specifically limited. In some embodiments, the virus-like particle may comprise or consist of Qβ protein, HBcAg or AP205.

In some embodiments, Qβ protein may have the amino acid sequence shown below:

MAKLETVTLGNIGKDGKQTLVLNPRGVNPTNGVASLSQAGAVPALEKRV
TVSVSQPSRNRKNYKVQVKIQNPTACTANGSCDPSVTRQAYADVTFSFTQ
YSTDEERAFVRTELAALLASPLLIDAIDQLNPAY (SEQ ID NO: 1)

In some embodiments, HBcAg may have the amino acid sequence shown below:

MDIDPYKEFGATVELLSFLPSDFFPSVRDLLDTASALYREALESPEHCSPH
HTALRQAILCWGELMTLATWVGNNLEDPASRDLVVNYVNTNMGLKIRQ
LLWFHISCLTFGRETVLEYLVSFGVWIRTPPAYRPPNAPILSTLPETTVVRR
RDRGRSPRRRTPSPRRRRSQSPRRRRSQSRESQC (SEQ ID NO: 2)

In some embodiments, AP205 may have the amino acid sequence shown below:

MANKPMQPITSTANKIVWSDPTRLSTTFSASLLRQRVKVGIAELNNVSGQ
YVSVYKRPAPKPEGCADACVIMPNENQSIRTVISGSAENLATLKAEWETH
KRNVDTLFASGNAGLGFLDPTAAIVSSDTTA (SEQ ID NO: 3)

In some embodiments of the methods described above, the multimer, such as virus-like particle, comprises or consists of the target antigen. In other words, at least one subunit of the multimer itself may serve as the target antigen. For example, the target antigen may be Qβ protein, HBcAg or AP205, or another virus-derived protein.

In other embodiments of the methods described above, the antigen complex comprises a loaded target antigen, which may be a bacteria- or virus-derived antigen. For example, the antigen selected from the bacteria- or virus-derived antigens listed in Table 3 above can be used.

In some embodiments, the bacteria- or virus-derived antigen may be a mycobacterium tuberculosis-derived antigen, for example, selected from crystallin and Rv3133c.

In some embodiments, the bacteria- or virus-derived antigen may be a superbacteria-derived antigen, for example, selected from Klebsiella pneumoniae carbapenemase and penicillin binding protein.

In some embodiments, the bacteria- or virus-derived antigen may be a lentivirus-derived antigen, for example, selected from HBV pre-S1 antigen and EBV LMP1 antigen.

In some other embodiments of the methods described above, the antigen complex comprises a loaded target antigen, which may be a tumor-associated antigen. The tumor-associated antigen used is not particularly limited, and may be any antigen associated with tumor development or aggressiveness. For example, the tumor-associated antigens as described in the above multimer-based antigen complex of the present disclosure can be used.

In some embodiments, the tumor antigen may be selected from Her2, p53, or tumor neoantigen.

In any embodiment of the methods described above, the method may be an in vitro method. For example, the isolated population of B cells may be contacted with a multimer-based antigen complex in vitro, such that the B cells recognize and process the antigen complex, and present the target antigen to CD4+ T cells, thereby activating CD4+ T cells and promoting differentiation of CD4+ T cells into Tfh and/or Th1 cells.

In other embodiments, some steps of the method may occur in vitro, while the rest occur in vivo. For example, the isolated population of B cells can be contacted with a multimer-based antigen complex in vitro, such that the B cells recognize and process the antigen complex, and present the complex of the target antigen and MHC II on the cell surface. Subsequently, the population of B cells can be administered to the subject, such that the population of B cells activate CD4+ T cells in the subject and promote differentiation of CD4+ T cells into Tfh and/or Th1 cells.

In still other embodiments, the method occurs in the subject in vivo. For example, by directly administering the multimer-based antigen complex of the present disclosure to the subject, it can be recognized by the population of B cells in the subject, thereby activating CD4+ T cells and promoting differentiation of the CD4+ T cells into Tfh and/or Th1. For example, the population of B cells may be a population of B cells naturally occurring in the subject, or a population of B cells transferred to the subject.

### Methods for preventing and/or treating a disease

In one aspect, the present disclosure provides a method for preventing and/or treating a disease in a subject in need thereof, the method comprising the step of administering to the subject an effective amount of a multimer-based antigen complex, wherein the multimeric antigen complex comprises:
i) a multimer assembled from a plurality of subunits; and
ii) an immunostimulant,
wherein the plurality of subunits comprise or consist of a target antigen, and wherein the immunostimulant is packaged in the multimer, or attached to the multimer by physical adsorption or chemical linkage;
alternatively, the antigen complex comprising:
i) a multimer assembled from a plurality of subunits;
ii) a loaded target antigen; and
iii) an immunostimulant,
wherein the target antigen is attached to the surface of the multimer by physical adsorption or chemical linkage, or fused with at least a part of the plurality of subunits by gene fusion, which fusion does not affect the assembly of the multimer, and the target antigen is displayed on the surface of the multimer after the multimer is assembled; and wherein the immunostimulant is packaged in the multimer, or attached to the multimer by physical adsorption or chemical linkage.

In another aspect, the present disclosure provides a method for preventing and/or treating a disease in a subject in need thereof, comprising:
a) isolating a population of B cells from the subject;
b) contacting a multimer-based antigen complex with the population of B cells;
   i) a multimer assembled from a plurality of subunits; and
   ii) an immunostimulant,
   wherein the plurality of subunits comprise or consist of a target antigen, and wherein the immunostimulant is packaged in the multimer, or attached to the multimer by physical adsorption or chemical linkage;
   alternatively, the antigen complex comprising:
   i) a multimer assembled from a plurality of subunits;
   ii) a loaded target antigen; and
   iii) an immunostimulant,
   wherein the target antigen is attached to the surface of the multimer by physical adsorption or chemical linkage, or fused with at least a part of the plurality of subunits by gene fusion, which fusion does not affect the assembly of the multimer, and the target antigen is displayed on the surface of the multimer after the multimer is assembled; and wherein the immunostimulant is packaged in the multimer, or attached to the multimer by physical adsorption or chemical linkage;
   wherein at least a part of the population of B cells are capable of recognizing at least one of the subunits;
c) incubating the antigen complex with the population of B cells to allow B cells to recognize and process the antigen complex, and present the target antigen on the cell surface; and
d) administering the population of B cells to the subject.

As described above, the multimer-based antigen complex of the present disclosure can be recognized and presented by B cells to activate CD4+ T cells and promote differentiation of CD4+ T cells into Tfh and/or Th1 cells. Due to the function of CD4+ T cells, especially Tfh and Th1, in immune response, especially in adaptive immune response, the method for activating CD4+ T cells and promoting their differentiation into Tfh and/or Th1 of the present disclosure can be used to prevent and/or treat a disease. Those skilled in the art will also understand that there is no restriction on types of diseases to be prevented and/or treated, as long as they are involved in immune responses in an organism, including innate immune responses and adaptive immune responses, and examples of diseases may particularly include various infectious diseases and cancers.

In some embodiments of the methods described above, the population of B cells is a population of B cells present in the subject. For example, the population of B cells may be a population of B cells naturally occurring in the subject, or a population of B cells transferred to the subject. In this case, the method for activating CD4+ T cells and the method for promoting differentiation of CD4+ T cells of the present disclosure can occur in the subject.

In other embodiments of the methods described above, the population of B cells may be a population of B cells isolated from peripheral blood or a lymphoid organ, such as thymus, spleen, and tonsils, of a donor.

In further embodiments of the method for preventing and/or treating a disease in a subject in need thereof as described above, after step c), the method further comprises a step of screening, enriching and/or amplifying the B cells that recognize the subunit. In other embodiments, after step c), the method may further comprise a step of screening the B cells that recognize the subunit, and introducing a gene sequence encoding an immunoglobulin receptor into the population of B cells, to increase the number of B cells that recognize the subunit in the population.

In any embodiment of the methods for preventing/treating a disease in a subject in need thereof as described above, the multimer may have a diameter of about 10 nm to about 1000 nm, for example, about 10 nm to about 500 nm, about 10 nm to about 300 nm, about 10 nm to about 200 nm, about 10 nm to about 100 nm, about 10 nm to about 50 nm, about 20 nm to about 1000 nm, about 20 nm to about 500 nm, about 20 nm to about 300 nm, about 20 nm to about 200 nm, about 20 nm to about 100 nm, about 20 nm to about 50 nm, about 50 nm to about 1000 nm, about 50 nm to about 500 nm, about 50 nm to about 300 nm, about 50 nm to about 200 nm, or about 50 nm to about 100 nm.

In any embodiment of the methods for preventing/treating a disease in a subject in need thereof as described above, the multimer may comprise at least 4 subunits, for example at least 10 subunits, at least 20 subunits, at least 50 subunits, at least 100 subunits, or at least 200 subunits. The multimer may have 10 to 1000 subunits, for example 20 to 500 subunits, 50 to 300 subunits, or 100 to 200 subunits.

In any embodiment of the method for preventing and/or treating a disease in a subject in need thereof as described above, the immunostimulant comprises a bacteria-derived ssRNA, an artificially synthesized ssRNA or a derivative thereof, an artificially synthesized CpG-containing oligonucleotide, an interferon, a cytokine, or any combination thereof. In some embodiments, the bacteria-derived ssRNA may be an E. coli-derived ssRNA. In some embodiments, the interferon may be selected from type I interferon, type II interferon, type III interferon, and a combination thereof. In some embodiments, the cytokine may be selected from IL-6, IL-12, IL21, and a combination thereof.

In some embodiments, one or more selected from the immunostimulants listed in Table 1 and Table 2 above, or other natural or artificial immunostimulants known in the art may be used.

In a further embodiment of the method described above, the multimer may be a virus-like particle, another natural multimer or an artificially synthesized multimer. The multimer may be assembled from multiple copies of one subunit, or may be assembled from multiple copies of two or more subunits. Selection of the multimer is not specifically limited. In some embodiments, the virus-like particle may comprise or consist of Qβ protein, HBcAg or AP205.

In some embodiments, Qβ protein may have the amino acid sequence shown below:

MAKLETVTLGNIGKDGKQTLVLNPRGVNPTNGVASLSQAGAVPALEKRV
TVSVSQPSRNRKNYKVQVKIQNPTACTANGSCDPSVTRQAYADVTFSFTQ
YSTDEERAFVRTELAALLASPLLIDAIDQLNPAY (SEQ ID NO: 1)

In some embodiments, HBcAg may have the amino acid sequence shown below:

MDIDPYKEFGATVELLSFLPSDFFPSVRDLLDTASALYREALESPEHCSPH
HTALRQAILCWGELMTLATWVGNNLEDPASRDLVVNYVNTNMGLKIRQ
LLWFHISCLTFGRETVLEYLVSFGVWIRTPPAYRPPNAPILSTLPETTVVRR
RDRGRSPRRRTPSPRRRRSQSPRRRRSQSRESQC (SEQ ID NO: 2)

In some embodiments, AP205 may have the amino acid sequence shown below:

MANKPMQPITSTANKIVWSDPTRLSTTFSASLLRQRVKVGIAELNNVSGQ
YVSVYKRPAPKPEGCADACVIMPNENQSIRTVISGSAENLATLKAEWETH
KRNVDTLFASGNAGLGFLDPTAAIVSSDTTA (SEQ ID NO: 3)

In some embodiments of the methods for preventing/treating a disease in a subject in need thereof as described above, the multimer, such as virus-like particle, comprises or consists of the target antigen. For example, the target antigen may be Qβ protein, HBcAg or AP205, or another virus-derived protein.

In other embodiments of the method for preventing and/or treating a disease in a subject in need thereof as described above, the disease is an infectious disease, and the antigen complex comprises a loaded target antigen that is a bacteria-derived or virus-derived antigen. For example, an antigen selected from the bacteria-derived or virus-derived antigens listed in Table 3 above can be used.

In some embodiments, the bacteria- or virus-derived antigen is a mycobacterium tuberculosis-derived antigen, for example, selected from crystallin and Rv3133c.

In some embodiments, the bacteria- or virus-derived antigen is a superbacteria-derived antigen, for example, selected from Klebsiella pneumoniae carbapenemase and penicillin binding protein.

In some embodiments, the bacteria- or virus-derived antigen is a lentivirus-derived antigen, for example, selected from HBV pre-S1 antigen and EBV LMP1 antigen.

In other embodiments of the methods described above, the disease is a cancer. Examples of the cancer include, but are not limited to: basal cell carcinoma, biliary tract cancer; bladder cancer; bone cancer; brain and CNS cancer; breast cancer; cancer of the peritoneum; cervical cancer; cholangiocarcinoma; choriocarcinoma; colon and rectum cancer; connective tissue cancer; cancer of the digestive system; endometrial cancer; esophageal cancer; eye cancer; cancer of the head and neck; gastric cancer (including gastrointestinal cancer); glioblastoma; hepatic carcinoma; hepatoma; intra-epithelial neoplasm; kidney or renal cancer; larynx cancer; leukemia; liver cancer; lung cancer (e.g. , small-cell lung cancer, non-small cell lung cancer, adenocarcinoma of the lung, and squamous carcinoma of the lung); lymphoma including Hodgkin's and non-Hodgkin's lymphoma; melanoma; myeloma; neuroblastoma; oral cavity cancer (e.g., lip, tongue, mouth, and pharynx); ovarian cancer; pancreatic cancer; prostate cancer; retinoblastoma; rhabdomyosarcoma; rectal cancer; cancer of the respiratory system; salivary gland carcinoma; sarcoma; skin cancer; squamous cell cancer; stomach cancer; teratocarcinoma; testicular cancer; thyroid cancer; uterine or endometrial cancer; cancer of the urinary system; vulval cancer; as well as other carcinomas and sarcomas; as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblasts leukemia; and post-transplant lymphoproliferative disorder (PTLD), as well as abnormal vascular proliferation associated with phakomatoses, edema (such as that associated with brain tumors), tumors of primitive origins and Meigs' syndrome.

In the case where the disease to be treated is a cancer, the loaded target antigen is a tumor-associated antigen, and the tumor-associated antigen used is not particularly limited, and may be any antigen associated with tumor development or aggressiveness. For example, the tumor-associated antigen as described in the above multimer-based antigen complex of the present disclosure can be used. In some embodiments, the tumor-associated antigen may be selected from Her2, p53 and tumor neoantigen.

Numerous tumor antigens have been defined in terms of various solid tumors: MAGE 1, 2 and 3, defined by immunity; MART-1/Melan-A, gp100, carcinoembryonic antigen (CEA), HER-2, mucin (i.e., MUC-1), prostate specific antigen (PSA) and prostatic acid phosphatase (PAP). In addition, viral proteins such as hepatitis B (HBV), Epstein-Barr (EBV) and human papilloma (HPV) have been shown to be important in the development of hepatocellular carcinoma, lymphoma and cervical cancer, respectively. However, tumors use or benefit from a range of different immune evasion mechanisms, such that the immune system of cancer patients often fail to respond to tumor antigens. Some examples of cancer antigens that are normally associated with spermatocytes or spermatogonia of the testis, placenta, and ovary include cancer-testis (CT) antigens BAGE, GAGE, MAGE-1 and MAGE-3, NY-ESO-1, SSX. These antigens are found in melanoma, lymphoma, lung cancer, bladder cancer, colon cancer and breast cancer. Tumor-associated antigens normally found in melanocytes, epithelial tissues, prostate and colon also include differentiation antigens Gp100, Melan-A/Mart-1, tyrosinase, PSA, CEA and Mammaglobin-A. These antigens are found in melanoma, prostate cancer, colon cancer and breast cancer. Some tumor-associated antigens are shared antigens that are ubiquitously expressed at low levels but overexpressed in cancers. Examples of overexpressed tumor-associated antigens include p53, HER-2/neu, livin and survivin found in esophagus, liver, pancreas, colon, breast, ovary, bladder, and prostate cancer. Other tumor-associated antigens are unique, such as β-catenin-m, β-actin/4/m, myosin/m, HSP70-2/m and HLA-A2-R170J, which are associated with one or more of melanoma, non-small cell lung cancer and kidney cancer. Other tumor-associated antigens are tumor-associated carbohydrate antigens normally found in epithelial tissues such as renal, intestinal, and colorectal tissues. These tumor-associated antigens include GM2, GD2, GD3, MUC-1, sTn, abd globo-H, which can be found in melanoma, neuroblastoma, colorectal cancer, lung cancer, breast cancer, ovarian cancer, and prostate cancer.

As used herein, the term "treat", "treatment" or "treating," "refers to therapeutic treatments in which the purpose is to reverse, alleviate, ameliorate, inhibit, slow down or stop the progression or severity of a condition associated with, a disease or disorder. The term "treating" includes reducing or alleviating at least one side effect or symptom of a disease or disorder. Treatment is generally "effective" if one or more symptoms or clinical markers are reduced. Alternatively, treatment is "effective" if the progression of a disease is reduced or halted. In other words, "treatment" includes not only the improvement of symptoms or markers, but also a cessation or at least slowing of progress or worsening of symptoms that would be expected in absence of treatment. Beneficial or desired clinical results include, but are not limited to, alleviation of one or more symptoms, diminishment of extent of disease, stabilized (i.e., not worsening) state of disease, delay or slowing of disease progression, amelioration or palliation of the disease state, and remission (whether partial or total), whether detectable or undetectable. The term "treatment" of a disease also includes alleviation of symptoms or side effects of the disease (including palliative treatment).

As used herein, the terms "subject" and "individual" are used interchangeably herein, and refer to an animal, and include mammals such as rat, mouse, rabbit, sheep, cat, dog, cow, pig, and non-human primate. The term "subject" also includes any vertebrate, including but not limited to mammals, reptiles, amphibians, and fish. However, advantageously, the subject is a mammal such as a human or other mammals, such as a domesticated mammal, e.g. dog, cat, horse, and the like. Production mammals, e.g. cow, sheep, pig, and the like are also included in the term subject.

In any embodiment of the methods for preventing and/or treating a disease as described above, the method may further comprise administering to the subject other therapies, such as anti-cancer therapy, chemotherapeutic, or immunomodulatory agent. In one embodiment, the immunomodulatory agent comprises an immune checkpoint inhibitor. In one embodiment, the immune checkpoint inhibitor binds to one or more of the following: PD1, PDL1, PDL2, CTLA4, LAG3, TIM3, TIGIT, and/or CD103. In one embodiment, the immune checkpoint inhibitor is a PD1, PDL1, and/or PDL2 inhibitory agent.

The term "anti-cancer therapy" refers to a therapy useful in treating cancer. Examples of anti-cancer therapeutic agents include, but are not limited to, e.g., surgery, chemotherapeutic agents, growth inhibitory agents, cytotoxic agents, radiotherapy and agents used in radiation therapy, anti-angiogenesis agents, apoptotic agents, anti-tubulin agents, and other agents to treat cancer, such as anti-HER-2 antibodies, anti-CD20 antibodies, an epidermal growth factor receptor antagonist, HER1/EGFR inhibitor, platelet derived growth factor inhibitors, a COX-2 inhibitor, interferons, cytokines, antagonists that bind to one or more of the following targets: PD1, PDL1, PDL2; CTLA4; LAG3; CD 103; TIM-3 and/or other ´HM family members; CEACAM-1 and/or other CEACAM family members, ErbB2, ErbB3, ErbB4, PDGFR-beta, BlyS, APRIL, BCMA or VEGF receptor(s), TRAIL/Apo2, and other bioactive and organic chemical agents, etc. Combinations thereof are also specifically contemplated for the methods described herein.

In some embodiments, an anti-cancer therapy comprises an immunotherapy such as adoptive cell transfer. "Adoptive cell transfer," as used herein, refers to immunotherapies involving genetically engineering a subject or patient's own T cells to produce special receptors on their surface called chimeric antigen receptors (CARs). CARs are proteins that allow the T cells to recognize a specific protein (antigen) on tumor cells. These engineered CAR T cells are then grown in the laboratory until they count in the billions. The expanded population of CAR T cells is then infused into the patient. After the infusion, the T cells multiply in the subject's body and, with guidance from their engineered receptor, recognize and kill cancer cells that harbor the antigen on their surfaces.

As used herein, the terms "chemotherapy" or "chemotherapeutic agent" refer to any chemical agent with therapeutic usefulness in the treatment of diseases characterized by abnormal cell growth. Such diseases include tumors, neoplasms and cancer as well as diseases characterized by hyperplastic growth. Chemotherapeutic agents as used herein encompass both chemical and biological agents. These agents function to inhibit a cellular activity upon which the cancer cell depends for continued survival. Categories of chemotherapeutic agents include alkylating/alkaloid agents, antimetabolites, hormones or hormone analogs, and miscellaneous antineoplastic drugs. Most if not all of these agents are directly toxic to cancer cells and do not require immune stimulation. In one embodiment, a chemotherapeutic agent is an agent of use in treating neoplasms such as solid tumors. In one embodiment, a chemotherapeutic agent is a radioactive molecule. One of skill in the art can readily identify a chemotherapeutic agent of use.

By "radiation therapy" is meant the use of directed gamma rays or beta rays to induce sufficient damage to a cell so as to limit its ability to function normally or to destroy the cell altogether. It will be appreciated that there will be many ways known in the art to determine the dosage and duration of treatment.

### Pharmaceutical composition and use

In one aspect, the present disclosure relates to a pharmaceutical composition comprising the multimer-based antigen complex of the present disclosure, and optionally one or more of other therapeutic agents and/or pharmaceutically acceptable carriers.

The pharmaceutical composition comprising a peptide of the present disclosure can be formulated by conventional formulation methods as needed. The pharmaceutical compositions of the present disclosure may comprise in addition to the peptide of the present disclosure, carriers, excipients and such commonly used in pharmaceuticals without particular limitations. Examples of carriers that can be used in pharmaceutical compositions of the present disclosure include sterilized water (for example, water for injection), physiological saline, phosphate buffer, phosphate buffered saline, Tris buffered saline, 0.3% glycine, culture fluid, and the like. Further, the pharmaceutical compositions of the present disclosure may comprise as needed stabilizers, suspensions, preservatives, surfactants, solubilizing agents, pH adjusters, aggregation inhibitors, and the like. The pharmaceutical compositions of the present disclosure can induce specific immunity against URLC10-expressing cancer cells, and thus can be applied for the purpose of cancer treatment or prevention (prophylaxis).

The phrase "pharmaceutically-acceptable carrier" means a pharmaceutically-acceptable material, composition or vehicle, such as a liquid or solid filler, diluent, excipient, solvent, medium, encapsulating material manufacturing aid (e.g., lubricant, talc magnesium, calcium or zinc stearate, or steric acid), or solvent encapsulating material, involved in maintaining the stability, solubility or activity of the LAP binder. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, methylcellulose, ethyl cellulose, microcrystalline cellulose and cellulose acetate; (4) powdered tragacanth; (5) malt; (6) gelatin; (7) excipients, such as cocoa butter and suppository waxes; (8) oils, such as peanut oil, cottonseed oil, safflower oil, sesame oil, olive oil, corn oil and soybean oil; (9) glycols, such as propylene glycol; (10) polyols, such as glycerin, sorbitol, mannitol and polyethylene glycol (PEG); (11) esters, such as ethyl oleate and ethyl laurate; (12) agar; (13) buffering agents, such as magnesium hydroxide and aluminum hydroxide; (14) alginic acid; (15) pyrogen-free water; (16) isotonic saline; (17) Ringer's solution; (19) pH buffered solutions; (20) polyesters, polycarbonates and/or polyanhydrides; (21) bulking agents, such as polypeptides and amino acids (22) serum component, such as serum albumin, HDL and LDL; (23) C2-C12 alcohols, such as ethanol; and (24) other non-toxic compatible substances employed in pharmaceutical formulations. Release agents, coating agents, preservative and antioxidants can also be present in the pharmaceutical formulation. The terms such as "excipient", "carrier", "pharmaceutically acceptable carrier" or the like are used interchangeably herein.

The pharmaceutical compositions of the present disclosure may also comprise an adjuvant known for effectively establishing cellular immunity. An adjuvant refers to a compound that enhances the immune response against an antigen that has immunological activity when administered together (or successively) with the antigen. Known adjuvants described in literatures, for example, Clin Microbiol Rev 1994, 7: 277-89, can be used. Examples of a suitable adjuvant include aluminum salts (aluminum phosphate, aluminum hydroxide, aluminum oxyhydroxide and such), alum, cholera toxin, Salmonella toxin, IFA (Incomplete Freund's adjuvant), CFA (Complete Freund's adjuvant), ISCOMatrix, GM-CSF and other immunostimulatory cytokines, oligodeoxynucleotide containing the CpG motif (CpG7909 and such), oil-in-water emulsions, Saponin or its derivatives (QS21 and such), lipopolysaccharide such as Lipid A or its derivatives (MPL, RC529, GLA, E6020 and such), lipopeptides, lactoferrin, flagellin, double-stranded RNA or its derivatives (poli IC and such), bacterial DNA, imidazoquinolines (Imiquimod, R848 and such), C-type lectin ligand (trehalose-6,6'-dibehenate (TDB) and such), CDld ligand (alpha-galactosylceramide and such), squalene emulsions (MF59, AS03, AF03 and such), PLGA, and such, without being limited thereto. The adjuvant may be contained in another container separate from the pharmaceutical composition comprising a peptide of the present disclosure in the kits comprising the pharmaceutical composition of the present disclosure. In this case, the adjuvant and the pharmaceutical composition may be administered to a subject in succession, or mixed together immediately before administration to a subject. Such kits comprising a pharmaceutical composition comprising a peptide of the present disclosure and an adjuvant are also provided by the present disclosure. When the pharmaceutical composition of the present disclosure is a freeze-dried formulation, the kit can further comprise a re-dissolving solution. Further, the present disclosure provides kits comprising a container that houses a pharmaceutical composition of the present disclosure and a container that stores an adjuvant. The kit can further comprise as needed a container that stores the re-dissolving solution.

These compositions according to the present disclosure can be administered by any common route, as long as the target tissue can be accessible through that route. Examples of suitable methods for administering the peptides or pharmaceutical compositions of the present disclosure include oral, epidermal, subcutaneous, intramuscular, intraosseous, peritoneal, and intravenous injections, as well as systemic administration or local administration to the vicinity of the targeted sites, but are not limited thereto.

In certain embodiments of the present disclosure, the pharmaceutical composition of the present disclosure is packaged together with or stored in the device for administration. Devices used for injectable preparations include, but are not limited to, injection ports, auto-injectors, injection pumps, and injection pens. Devices used for atomized or powdered preparations comprise, but are not limited to, inhalers, insufflators, aspirators, and the like. Therefore, the present disclosure comprises an administration device comprising the pharmaceutical composition of the present disclosure for use in the treatment or prevention of one or more disorders described herein.

In another aspect, the present disclosure relates to use of the multimer-based antigen complex or pharmaceutical composition of the present disclosure in activation of CD4+ T cells and/or promotion of differentiation of CD4+ T cells into Tfh and/or Th1 cells.

In yet another aspect, the present disclosure relates to use of the multimer-based antigen complex or pharmaceutical composition of the present disclosure in prevention and/or treatment of a disease. In some embodiments, the disease is selected from an infectious disease and a cancer, for example, the infectious disease and cancer as described above with respect to methods for preventing and/or treating a disease.

In one aspect, the present disclosure relates to use of the multimer-based antigen complex of the present disclosure in the manufacture of a pharmaceutical composition for activating CD4+ T cells and/or promoting differentiation of CD4+ T cells into Tfh and/or Th1 cells.

In another aspect, the present disclosure relates to use of the multimer-based antigen complex of the present disclosure in manufacture of a pharmaceutical composition for treating a disease. In some embodiments, the disease is selected from an infectious disease and a cancer, for example, the infectious disease and cancer as described above with respect to methods for preventing and/or treating a disease.

### EXAMPLES

The embodiments of the present disclosure are further illustrated with reference to the following examples. However, it should be noted that these examples are as illustrative as the above embodiments and should not be construed as limiting the scope of the present disclosure in any way.

### Example 1: Multimer-based antigen complex is capable of robust activation of CD4+ T cells

Bacterial phage Qβ-derived VLPs (Qβ-VLPs) are assembled from single type of monomers and contains nucleic acids inside. Previous studies demonstrated that Qβ-VLPs induce robust antibody responses, including a GC response in the absence of any conventional adjuvants (Gatto, D. et al., (2004). Rapid response of marginal zone B cells to viral particles. J Immunol 173, 4308-4316; Liao, W. et al., (2017). Characterization of T-Dependent and T-Independent B Cell Responses to a Virus-like Particle. J Immunol 198, 3846-3856). The strong immunogenicity of Qβ-VLPs is known to rely heavily on their encapsulated nucleic acids as immunostimulant that may be either ssRNA derived from the host bacteria or CpG containing oligodeoxynucleotides (CpG ODN) artificially synthesized, which serve as TLR7 or TLR9 ligands to enhance the immune response (Jegerlehner, A. et al., (2007). TLR9 signaling in B cells determines class switch recombination to IgG2a. J Immunol 178, 2415-2420).

To explore how CD4+ T cells are activated in response to Qβ-VLPs, we generated VLPs assembled from both Qβ protein and a fusion protein of Qβ and ovalbumin-derived peptide, which can be recognized by CD4+ TCR transgenic T cells (OT-II). About 10-15% of the 180 monomers in the assembled VLPs are replaced with the fusion protein, which corresponds to about 20-30 copies of the OT-II CD4+ T cell epitopes in each particle. This type of VLPs generated was named Qβ-Ova.

To explore how Qβ-VLP activates CD4+ T cells, we adoptively transferred 5x10⁵ CFSE-labeled naive OT-II CD4+ T cells (CD4+ CD44lo CD62Lhi) into wild-type (WT) mice, followed by intraperitoneal immunization with Qβ-Ova one day later. Subsequently, the mice were sacrificed and their splenocytes were isolated for flow cytometry. The OT-II donor-derived CD4+ T cells were gated as Thy1.1+ from the total CD4+ T cells of spleen. The result showed that at different time points after immunization, OT-II CD4+ T cells showed a robust expansion (FIG. 2A), which was consistent with the extensive CFSE dilution in these cells (FIG. 2B). In this result, the CFSE dilution was quantified as proliferation index, which reflected the average number of cell divisions after immunization.

In addition to cell proliferation, we evaluated markers of T cell activation, including CD44 up-regulation and CD62L down-regulation (CD44 hi and CD62 lo). As shown in the result in FIG. 2C, up-regulation of CD44 and down-regulation of CD62L were observed in most of the CFSE-diluted (i.e. proliferated) cells, indicating the activation of these T cells.

In order to further determine whether Qβ-VLP immunization caused the differentiation of CD4+ T cells, we examined molecular markers related to the differentiation of different Th lineages in OT-II CD4+ T cells. The result showed that after using Qβ-VLP, both CXCR5 and PD-1 were dramatically up-regulated in OT-II CD4+ T cells, and Bcl-6 was also significantly up-regulated in a fraction of OT-II CD4+ T cells (FIG. 3A), indicating their differentiation toward Tfh lineage. In addition, T-bet, the transcription factor for Th1 differentiation, along with its target gene CXCR3, were also induced significantly in a large fraction of OT-II CD4+ T cells, indicating that Qβ-Ova also promoted the differentiation of CD4+ T cells into Th1 cells (FIG. 3B).

### Example 2. TLR signals in B cells rather than those in DCs are required for the activation and differentiation of CD4+ T cells induced by Qβ-VLPs

We further studied the function of B cells in the activation and differentiation of OT-II CD4+ T cells upon Qβ-VLP immunization. Specifically, we investigated whether the deficiency of MyD88, the adaptor protein downstream of TLR signaling, in B cells affects the activation of OT-II CD4+ T cells upon Qβ-VLP immunization. For this purpose, we used mice lacking MyD88 in B cells (MyD88^{f1/f1} CD79a-Cre, referred as B-MyD88 -/-). The result showed that at d3 post-immunization, there was a significant reduction of the OT-II CD4+ T cell expansion, with a significantly reduced proliferation index in the B-MyD88-/- mice compared with WT mice (FIG. 4A). More importantly, there was a dramatic defect in the induction of both Tfh and Th1 marker molecules in the CFSE-diluted cells in the B-MyD88-/- mice (FIG. 4B). The above result indicated that TLR signaling in B cells was required for Qβ-Ova-induced CD4+ T cell activation and differentiation.

In order to determine whether this effect of B cells on antigen-induced B cell activation depends on the antigen forms, we immunized mice with a mixture of soluble Ova and CpG ODN after OT-II CD4+ T cell transfer, and as described above, detect whether the markers for T cell activation and differentiation were induced in OT-II CD4+ T cells. There was no significant difference in OT-II CD4+ T cell proliferation or cell differentiation between WT and B-MyD88 -/mice (FIGs. 5A and 5B). This result indicated that in the case of using soluble antigens, TLR signals in B cells were not required to induce CD4+ T cell activation.

Since it is currently known that DCs are widely implicated in the initiation of CD4+ T cell responses (Iwasaki, A., and Medzhitov, R. (2010). Regulation of adaptive immunity by the innate immune system. Science 327, 291-295), we further studied whether TLR signaling in DCs can also promote the activation of CD4 T cells induced by Qβ-Ova. Surprisingly, it was found that compared with wild-type mice, mice lacking MyD88 in DCs (MyD88^{f1/f1} CD11c-Cre, referred as DC-MyD88-/-) exhibited no defect in OT-II CD4+ T cell proliferation and differentiation upon Qβ-Ova immunization (FIGs.6A and 6B). The above results indicated that the TLR signals in DCs were not required for inducing CD4+ T cell activation.

### Example 3. Mice lacking Qβ-specific B cells were unable to initiate CD4+ T cell activation induced by Qβ-VLP immunization.

The fact that Qβ-Ova elicited TLR signals in B cells instead of DCs to promote the activation and differentiation of CD4+ T cells led us to consider whether B cells were directly involved in the activation of CD4+ T cells during the early T cell response. Unlike DCs, B cells bind to antigens through specific B cell antigen receptors (BCRs). Therefore, we tested whether mice lacking BCRs that can specifically bind to Qβ-VLP had any defect in Qβ-Ova-induced CD4+ T cell activation and differentiation. In this experiment, we used BCR transgenic mice (MD4) previously found, which express BCRs that recognize egg lysozyme (Goodnow, CC et al., (1988). Altered immunoglobulin expression and functional silencing of self-reactive B lymphocytes in transgenic mice. Nature 334, 676-682), and contain very few Qβ specific B cells (Liao, W. et al., (2017). Characterization of T-Dependent and T-Independent B Cell Responses to a Virus -like Particle. J Immunol 198, 3846-3856).

We transferred naive OT-II CD4+ T cells into MD4 mice, followed by immunization with Qβ-Ova, and assessment of T cell activation and differentiation at day 3 post-immunization. Surprisingly, upon immunization with Qβ-Ova, there was a severe defect in the OT-II CD4+ T cell response in MD4 mice. Specifically, the proliferation index and the percentage of OT-II CD4+ T cells in MD4 mice were much lower than that in WT mice, indicating a severe defect in cell proliferation (FIG. 7A). In addition, very few OT-II CD4+ T cells in MD4 mice exhibited up-regulation of CD4+ T cell differentiation markers in response to Qβ-Ova immunization (FIGs. 7B and 7C).

In order to determine whether MD4 mice may harbor any other factors that affect CD4+ T cell activation, we immunized MD4 mice with a mixture of soluble Ova and CpG ODN after OT-II CD4+ T cell transfer. The result showed that, compared with the wild-type mice, there was no obvious defect in the activation of CD4+ T cells in response to soluble Ova in MD4 mice (FIGs. 7A-7C). This result indicated that in the case of immunization using a soluble antigen, the activation of CD4+ T cells does not depend on the presence of antigen-specific B cells. Since other APCs such as DCs are fully functional in MD4 mice, it is speculated that they can initiate CD4+ T cell activation induced by a soluble antigen.

In summary, antigen-specific B cells were required for the activation and differentiation of CD4+ T cells induced by a multimer-based antigen complex such as Qβ-Ova.

### Example 4. DCs were not required for CD4+ T cell activation induced by QP-VLP

To further prove that Qβ-VLP-induced CD4+ T cell activation depends on B cells rather than DCs, we generated chimeric mice with bone marrow (BM) from CD11c-DTR/GFP mice. CD11c-DTR/GFP mice express the fusion protein of diphtheria toxin receptor and GFP under the control of CD11c promoter (Jung, S. et al., (2002). In vivo depletion of CD11c+ dendritic cells abrogates priming of CD8+ T cells by exogenous cell-associated antigens. Immunity 17, 211-220). In these chimeric mice, the diphtheria toxin receptor was expressed on the cell surface of DCs. At the time of OT-II T cell transfer, the chimeras were treated with diphtheria toxin followed by immunization one day later. This treatment effectively depleted the DCs in the chimeric mice. At 24 hours post-immunization, we examined the activation of T cells in the chimeric mice and found that the markers for T cell activation, including CD69, CD62L and CD25, showed a dramatic change (FIG. 8). This result unexpectedly indicated that the depletion of DCs had no significant effect on CD4+ T cell activation, indicating that DCs were not required for Qβ-VLP immunization-induced CD4+ T cell activation. To confirm the depletion of DCs by diphtheria toxin treatment in the above chimeric mice, we tested CD4+ T cell activation induced by soluble Ova immunization. As expected, when DCs were depleted, the soluble antigen-induced CD4+ T cell activation was greatly suppressed.

### Example 5. Qβ-VLPs were captured by antigen-specific B cells effectively in vivo.

The above results strongly indicated that antigen-specific B cells functioned in CD4+ T cell activation induced by multimer-based antigen complexes. We further studied how such multimer-based antigen complexes are captured by B cells in vivo. To follow how Qβ-VLPs are bound and recognized by antigen-presenting cells after immunization, we injected AF647-labeled Qβ-VLP (Qβ-AF647) or PBS intravenously into WT mice, and sacrificed the mice 3 hours after injection and performed a spleen test.

The result showed that very low percentage of DCs in the spleen exhibited Qβ-AF647 binding after injection (FIG. 9A). By examining Qβ-AF647+ cells within MHCII+ cells (including B cells and DCs), we found that the vast majority of Qβ-AF647+ MHCII+ cells were B cells instead of DCs in WT mice (FIG. 9B). In addition, about 5% within Qβ-AF647+ MHCII+ B cells exhibited a high level of Qβ-AF647 binding. This high level of Qβ-AF647 binding was mediated by specific BCRs, since these high-level Qβ-AF647-bound cells were absent in MD4 mice (FIG. 9B).

We next examined whether B cells that showed high level of Qβ-AF647 binding exhibited features in favor of antigen presentation. We enriched these cells. Specifically, splenocytes from mice injected with Qβ-AF647 were incubated with FITC-labeled Qβ-VLP (Qβ-FITC), and then incubated with anti-FITC-conjugated magnetic beads to enrich Qβ+ B cells. Within the enriched cells, a large fraction exhibited high level of binding to Qβ-AF647 (FIG. 9C). More notably, most of these Qβ-AF647+ B cells from the enriched fraction were CD83+ (FIG. 9C). CD83 is a molecule that is upregulated upon B cell activation and is involved in the post-translational regulation of MHC II (Tze, LE et al., (2011). CD83 increases MHC II and CD86 on dendritic cells by opposing IL-10-driven MARCH1- mediated ubiquitination and degradation. J Exp Med 208, 149-165). The above results indicated that Qβ-VLP can effectively activate antigen-specific B cells. In addition, Qβ+ B cells enriched from mice immunized for 24 hours exhibited significant up-regulation of the costimulatory molecule CD86 and chemotaxis receptor CCR7 (FIG. 10), indicating that their capacity to stimulate T cells and to migrate to T cell zones was enhanced.

### Example 6. Antigen presentation by B cells was required for Qβ-VLP-induced CD4 T cell activation

The above results strongly suggested the capability of activated Qβ-specific B cells acting as antigen presenting cells (APCs) to activate T cells. In order to further evaluate the importance of cognate T cell-B cell interaction in the activation of CD4+ T cells upon Qβ-Ova immunization, we generated mice selectively deleted of MHC II in B cells (B-MHCII-/-) by transplanting mixed bone marrow cells from MHC II-/- (20%) and µMT (80%) mice to lethally irradiated WT mice. As we expected, the cell expansion of OT-II CD4+ T cells transferred to B-MHCII -/- mice in response to Qβ-Ova immunization was significantly reduced (FIG. 11). The above results demonstrated that the cognate interactions between antigen-specific B cells and CD4+ T cells contributed dominantly to the activation of CD4+ T cells induced by Qβ-VLP.

### Example 7. Antigen-specific B cells were involved in CD4+ T cell activation induced by influenza viruses

The above examples demonstrate that B cells play an important role in T cell activation and differentiation induced by a multimer-based antigen complex. We hypothesized that this mechanism might be an evolutionarily conserved pathway for defensing viruses, especially during viremia. In the case of viremia, viral antigens can travel directly to the spleen and reach B cell follicles through the sinuses and marginal zones. Therefore, antigen-specific B cells, which are also equipped with TLRs for sensing viral nucleotides, may be extremely sensitive to pathogens in the blood and are responsible for the initiation of the adaptive immune system. To test this hypothesis, we used a modified strain of influenza A virus carrying the OT-II CD4+ T cell epitope (PR8-OVA) (Hua, L. et al., (2013). Cytokine-dependent induction of CD4+ T cells with cytotoxic potential during influenza virus infection. J Virol 87, 11884-11893) for immunization. After OT-II CD4+ T cells were transferred to WT or MD4 mice, formalin-inactivated PR8-OVA was injected intraperitoneally into the mice. The results showed that, compared with WT mice, MD4 mice exhibited a significant reduction in CD4+ T cell expansion and differentiation into helper T upon PR8-OVA immunization (FIGs. 12A and 12B). To test whether B cells are sufficient to initiate CD4+ T cell activation in the absence of DCs, we immunized CD11c-DTR/GFP chimeric mice with inactivated PR8-OVA after OT-II CD4 T cell transfer and diphtheria toxin treatment. There was no difference in CD4+ T cell activation between the control and diphtheria toxin-treated mice (FIG. 12C), indicating that DCs were not essential for the CD4+ T cell response induced by influenza A virus.

Without departing from the scope and spirit of the present disclosure, various modifications and changes of the method and system described in the present disclosure are apparent to those skilled in the art. Although the present disclosure has been described in connection with specific preferred embodiments, it should be understood that the claimed disclosure should not be unduly limited to these specific embodiments. In fact, various modifications of the described modes for implementing the present disclosure that are obvious to those skilled in molecular biology, immunology, or related fields are intended to fall within the scope of the appended claims.

## Claims

1. A method for activating CD4+ T cells, comprising the following steps:
a) contacting a multimer-based antigen complex with a population of B cells,
the antigen complex comprising:
i) a multimer assembled from a plurality of subunits; and
ii) an immunostimulant,
wherein the plurality of subunits comprise or consist of a target antigen, and wherein the immunostimulant is packaged in the multimer, or attached to the multimer by physical adsorption or chemical linkage;
alternatively, the antigen complex comprising:
i) a multimer assembled from a plurality of subunits;
ii) a loaded target antigen; and
iii) an immunostimulant,
wherein the target antigen is attached to the surface of the multimer by physical adsorption or chemical linkage, or fused with at least a part of the plurality of subunits by gene fusion, which fusion does not affect the assembly of the multimer, and the target antigen is displayed on the surface of the multimer after the multimer is assembled; and wherein the immunostimulant is packaged in the multimer, or attached to the multimer by physical adsorption or chemical linkage,
wherein at least a part of the population of B cells are capable of recognizing at least one of the subunits;
b) incubating the antigen complex with the population of B cells to allow B cells to recognize and process the antigen complex, and present the target antigen on the cell surface; and
c) contacting the population of B cells with CD4+ T cells to activate the CD4+ T cells.

2. A method for promoting differentiation of CD4+ T cells into follicular helper T cells (Tfh) and/or helper T cells 1 (Th1), comprising the following steps:
a) contacting a multimer-based antigen complex with a population of B cells,
the antigen complex comprising:
i) a multimer assembled from a plurality of subunits; and
ii) an immunostimulant,
wherein the plurality of subunits comprise or consist of a target antigen, and wherein the immunostimulant is packaged in the multimer, or attached to the multimer by physical adsorption or chemical linkage;
alternatively, the antigen complex comprising:
i) a multimer assembled from a plurality of subunits;
ii) a loaded target antigen; and
iii) an immunostimulant,
wherein the target antigen is attached to the surface of the multimer by physical adsorption or chemical action, or fused with at least a part of the plurality of subunits by gene fusion, which fusion does not affect the assembly of the multimer, and the target antigen is displayed on the surface of the multimer after the multimer is assembled; and wherein the immunostimulant is packaged in the multimer, or attached to the multimer by physical adsorption or chemical action,
wherein at least a part of the population of B cells are capable of recognizing at least one of the subunits;
b) incubating the antigen complex with the population of B cells to allow B cells to recognize and process the antigen complex, and present the target antigen on the cell surface; and
c) contacting the B cells with CD4+ T cells to promote differentiation of the CD4+ T cells into Tfh and/or Th1.

3. The method according to claim 1 or 2, wherein the population of B cells is a population of B cells isolated from peripheral blood or a lymphoid organ of a donor.

4. The method according to any one of claims 1 to 3, wherein after step b), the method further comprises a step of screening, enriching and/or amplifying the B cells that recognize the subunit.

5. The method according to any one of claims 1 to 3, wherein after step b), the method further comprises a step of screening the B cells that recognize the subunit, and introducing a gene sequence encoding an immunoglobulin receptor into the population of B cells, to increase the number of B cells that recognize the subunit in the population.

6. The method according to any one of claims 1 to 5, wherein the multimer has a diameter of 10 nm to 1000 nm.

7. The method according to any one of claims 1 to 6, wherein the multimer comprises at least 4 subunits.

8. The method according to any one of claims 1 to 7, wherein the immunostimulant is selected from a bacteria-derived ssRNA, an artificially synthesized ssRNA or a derivative thereof, an artificially synthesized CpG-containing oligonucleotide, an interferon, a cytokine, and a combination thereof.

9. The method according to claim 8, wherein the bacteria-derived ssRNA is an E. coli-derived ssRNA.

10. The method according to claim 8, wherein the interferon is selected from type I interferon, type II interferon, type III interferon, and a combination thereof.

11. The method according to claim 8, wherein the cytokine is selected from IL-6, IL-12, IL21, and a combination thereof.

12. The method according to any one of claims 1 to 11, wherein the multimer is a virus-like particle.

13. The method according to claim 12, wherein the virus-like particle comprises or consists of Qβ protein, HBcAg or AP205.

14. The method according to claim 12 or 13, wherein the virus-like particle comprises or consists of the target antigen.

15. The method according to any one of claims 1 to 13, wherein the antigen complex comprises a loaded target antigen that is a bacteria-derived or virus-derived antigen.

16. The method according to claim 15, wherein the target antigen is a mycobacterium tuberculosis-derived antigen.

17. The method according to claim 16, wherein the target antigen is selected from crystallin and Rv3133c.

18. The method according to claim 15, wherein the target antigen is a superbacteria-derived antigen.

19. The method according to claim 18, wherein the target antigen is selected from Klebsiella pneumoniae carbapenemase and penicillin binding protein.

20. The method according to claim 15, wherein the target antigen is a lentivirus-derived antigen.

21. The method according to claim 20, wherein the target antigen is selected from HBV pre-S1 antigen and EBV LMP1 antigen.

22. The method according to any one of claims 1 to 12, wherein the antigen complex comprises a loaded target antigen that is a tumor-associated antigen.

23. The method according to claim 22, wherein the tumor-associated antigen is selected from Her2, p53 and tumor neoantigen.

24. The method according to any one of claims 1 to 23, wherein the method is an in vitro method.

25. The method according to any one of claims 1 to 23, wherein step c) of the method occurs in vivo.

26. A method for preventing and/or treating a disease in a subject in need thereof, comprising:
a) isolating a population of B cells from the subject;
b) contacting a multimer-based antigen complex with the population of B cells;
the antigen complex comprising:
i) a multimer assembled from a plurality of subunits; and
ii) an immunostimulant,
wherein the plurality of subunits comprise or consist of a target antigen, and wherein the immunostimulant is packaged in the multimer, or attached to the multimer by physical adsorption or chemical linkage;
alternatively, the antigen complex comprising:
i) a multimer assembled from a plurality of subunits;
ii) a loaded target antigen; and
iii) an immunostimulant,
wherein the target antigen is attached to the surface of the multimer by physical adsorption or chemical linkage, or fused with at least a part of the plurality of subunits by gene fusion, which fusion does not affect the assembly of the multimer, and the target antigen is displayed on the surface of the multimer after the multimer is assembled; and wherein the immunostimulant is packaged in the multimer, or attached to the multimer by physical adsorption or chemical linkage;
wherein at least a part of the population of B cells are capable of recognizing at least one of the subunits;
c) incubating the antigen complex with the population of B cells to allow B cells to recognize and process the antigen complex, and present the target antigen on the cell surface; and
d) administering the population of B cells to the subject.

27. The method according to claim 26, wherein the population of B cells is a population of B cells isolated from peripheral blood or a lymphoid organ of the subj ect.

28. The method according to claim 26 or 27, wherein after step c), the method further comprises a step of screening, enriching and/or amplifying the B cells that recognize the subunit.

29. The method according to any one of claims 26 to 28, wherein after step c), the method further comprises a step of screening the B cells that recognize the subunit, and introducing a gene sequence encoding an immunoglobulin receptor into the population of B cells, to increase the number of B cells that recognize the subunit in the population.

30. The method according to any one of claims 26 to 29, wherein the multimer has a diameter of 10 nm to 1000 nm.

31. The method according to any one of claims 26 to 30, wherein the multimer comprises at least 4 subunits.

32. The method according to any one of claims 26 to 31, wherein the immunostimulant comprises a bacteria-derived ssRNA, an artificially synthesized ssRNA or a derivative thereof, an artificially synthesized CpG-containing oligonucleotide, an interferon, a cytokine, or a combination thereof.

33. The method according to claim 32, wherein the bacteria-derived ssRNA is an E. coli-derived ssRNA.

34. The method according to claim 32, wherein the interferon is selected from type I interferon, type II interferon, type III interferon, and a combination thereof.

35. The method according to claim 32, wherein the cytokine is selected from IL-6, IL-12, IL21, and a combination thereof.

36. The method according to any one of claims 26 to 35, wherein the multimer is a virus-like particle.

37. The method according to claim 36, wherein the virus-like particle comprises or consists of Qβ protein, HBcAg or AP205.

38. The method according to claim 36 or 37, wherein the virus-like particle comprises or consists of the target antigen.

39. The method according to any one of claims 26 to 37, wherein the disease is an infectious disease, and the antigen complex comprises a loaded target antigen that is a bacteria-derived or virus-derived antigen.

40. The method according to claim 39, wherein the target antigen is a mycobacterium tuberculosis-derived antigen.

41. The method according to claim 40, wherein the target antigen is selected from a crystallin and Rv3133c.

42. The method according to claim 39, wherein the target antigen is a superbacteria-derived antigen.

43. The method according to claim 42, wherein the target antigen is selected from Klebsiella pneumoniae carbapenemase and penicillin binding protein.

44. The method according to claim 39, wherein the target antigen is a lentivirus-derived antigen.

45. The method according to claim 44, wherein the target antigen is selected from HBV pre-S1 antigen and EBV LMP1 antigen.

46. The method according to any one of claims 26 to 36, wherein the disease is a cancer, and the antigen complex comprises a loaded target antigen that is a tumor-associated antigen.

47. The method according to claim 46, wherein the tumor-associated antigen is selected from Her2, p53 and tumor neoantigen.
